# EUROPÄISCHE PATENTSCHRIFT

(11) **EP 3 318 291 B1**
(45) Veröffentlichungstag und Bekanntmachung des Hinweises auf die Patenterteilung: **25.12.2024**
(21) Anmeldenummer: 16197046.2
(22) Anmeldetag: 03.11.2016
(51) Int. Cl.: A61M 1/00

(54) **FLÜSSIGKEITSMANAGEMENT IN EINER OPHTHALMOLOGISCHEN APPARATUR**
FLUID MANAGEMENT IN AN OPHTHALMOLOGICAL APPARATUS
GESTION DE LIQUIDE DANS UN APPAREIL OPHTALMOLOGIQUE

(43) Veröffentlichungstag der Anmeldung: 09.05.2018
(73) Patentinhaber: This AG, 9435 Heerbrugg (CH)
(72) Erfinder: KÖPPEL, Thomas, 9443 Widnau (CH)
(74) Vertreter: Kaminski Harmann

(56) Entgegenhaltungen:
- US-A1- 2011 054 385
- US-A1- 2011 106 004
- US-A1- 2012 215 160
- US-A1- 2014 163 455
- US-A1- 2014 323 953
- US-A1- 2014 328 697
- US-B1- 6 283 937

## Beschreibung

Die Erfindung betrifft ein ophthalmologisches Gerät nach dem Oberbegriff des Anspruchs 1 sowie ein Wechselteil für ein ophthalmologisches Gerät nach dem Oberbegriff des Anspruchs 10 als auch ein zugehöriges Verfahren.

Die Erfindung betrifft augenchirurgische Apparaturen. Bei Eingriffen innerhalb des Auges, erfolgt dabei in vielen Fällen eine Aspiration, also Absaugung von Augenflüssigkeit respektive von bei der Operation abgetragenen Partikeln. Zum Beispiel wird im Rahmen einer Kataraktoperation die getrübte Linse aus dem Auge entfernt und durch eine Intra-okularlinse(IOL)ersetzt. Beispielsweise wird dabei eine Phakoemulsifikation (Linsenkernverflüssigung) durchgeführt, bei welcher über einen wenige Millimeter langen Schnitt im Bereich des Übergangs von Hornhaut zu Lederhaut Operationswerkzeuge ins Innere des Auges eingeführt werden. Es wird ein Stück der Kapsel entfernt und im Anschluss der Linseninhalt, z.B. mit einem Ultraschallgerät oder Laser, zerkleinert und entfernt. Das Entfernen der Linsenpartikel erfolgt dabei über eine an das Operationswerkzeug angeschlossenes Saugspülung, welches ein Kernpunkt der vorliegenden Erfindung darstellt.

Diese Saugspühlung aspiriert die zertrümmerten Linsenkern-Teilchen und ersetzt das Abgesaugte durch Flüssigkeitsinfusion, beispielsweise durch eine Balanced Salt Solution (BSS). Nach dem Entfernen des alten Linsenkerns wird durch die Öffnung eine zusammengefaltete, elastische Ersatzlinse in den Kapselsack eingebracht. Die Saugspülung wird von einer ophthalmologischen Apparatur, also von einem sich im Operationssaal befindlichen Operationsgeräts welches von Arzt und/oder Schwester bedient wird, bereitgestellt - meist gemeinsam mit weiteren für die Operation erforderlichen Funktionen. Neben der oben beschriebenen Phakoemulsifikation kommen dieselben medizinische Geräte mit Saugspülfunktion, welche diese Erfindung behandelt (bzw. allenfalls spezifische Ausführungsformen derartiger Geräte) auch bei anderen ophthalmologischen Eingriffen zum Einsatz, bei welchen eine interokulare Infusion, oft (aber nicht zwingend) gemeinsam mit einer Aspiration durchgeführt wird, beispielsweise bei Vitrektomie, Diathermie, Kapsulotomie, Glaukom-Chirurgie, Refraktive Chirurgie, Femto-Laser, etc. Beispielhaft beschreiben die Dokumente DE 696 21 562, US 2016/045367, WO 02/056850, US 2012/0215160, US 2014/0328697, US 2011/0054385, US 2014/0323953, US 2014/016345, US 6,283,937, US 2011/0106004 exemplarische Ausführungen derartiger medizinischer Gerätschaften.

US 2012/215160 offenbart ein ophthalmologisches System zur interokularen Druckstabilisierung mit einem Drucksensor direkt im Handstück beim Auge des Patienten. Dieser direkt im Auge gemessene Druck wird durch Ansteuerung eines speziell dafür ausgebildeten Aktuators im Gerät stabilisiert.

US 2014/328697 offenbart eine Multisegment-Peristaltikpumpe in einer medizinischen Kassette, deren Segmente zur Ripple-Kompensation Phasenversetzt angeordnet sind.

US 2011/054385 offenbart ein ophthalmologisches System mit einer ersten Irrigationsflüssigkeitsquelle sowie einer zweiten Irrigationsflüssigkeitsquelle, welche zweite Quelle bei Bedarf zugeschaltet werden kann.

US 2014/323953 offenbart ein augenchirurgisches System, bei welchem eine Einrichtung zum zumindest teilweisen ablassen des Vakuumdrucks vorgesehen ist.

US 2014/163455 offenbart ein Handstück zur Phakoemulsifikation, bei welchem eine Pumpe für die Irrigationsflüssigkeit direkt im Gehäuse des Handstücks angeordnet ist.

Um Sterilität zu gewährleisten, muss - insbesondere bezüglich der aspirierten Flüssigkeit, welche potentiell kontaminiert ist - eine intraokulare Infektionen oder Übertragung von Patient zu Patient sicher vermieden werden. Dies kann unter anderem beispielsweise durch Austausch eines Wechseleinsatzes, welcher meist als Kassette bezeichnet wird, erzielt werden. Diese Kassetten können als Einwegkassetten, Tageskassetten und/oder auch mehrfach sterilisierbar ausgeführt sein. Die Kassette weist dabei insbesondere jene Teile der Saugspühlung auf, welche mit potentiell kontaminierter Patientenflüssigkeit in Berührung gelangen oder gelangen könnten. Speziell können diese Teile etwa Pump- bzw. Absaugvorrichtungen, Ventile und/oder Sensoren für Druck und Unterdruck, Luftblasen, Durchfluss, etc. sein. Auch - vorzugsweise gegen Vertauschung mechanisch codierte - Anschlüsse zu Leitungen von und/oder zum Patienten, sowie für Anschlüsse für Abfall- und/oder Infusionsbehälter, etc. sind meist Teil einer derartigen austauschbaren Kassette, welche in austauschbarer Weise zumindest teilweise an oder im medizinischen Gerät angebracht werden. Die Dokumente DE 693 09 757, DE 693 21 264, DE 696 15 507, DE 696 15 633, DE 697 09 192, US 5,163,900, US 5,282,787, US 5,582,601, US 5,800,396, US 5,897,524, US 5,899,674, WO 1987/001943, WO 2004/108189, oder WO 2004/110524 zeigen artverwandte Kassetten.

Vorzugsweise werden Sensorik, Aktoren, Elektronik und/oder Mechanik möglichst im medizinischen Gerät und nicht in der austauschbaren Kassette untergebracht. Die Kassette bildet dabei also insbesondere nur die mit potentiell kontaminiertem Fluid in Kontakt tretender Teile der Sensorik und der Aktoren aus, also z.B. Messkammer, Membrane, Pump- oder Saugeinrichtungen, Quetschbereiche von Ventilen, etc. - also insbesondere passive Teile. Die aktiven Teile von Sensorik und Aktoren, insbesondere wenn diese einen beträchtlichen Kostenfaktor darstellen, werden vorzugsweise im medizinischen Gerät untergebracht.

Für die Saugspülfunktion, also für die Infusion und/oder Aspiration - oder anders ausgedrückt für die Zu- und Abfuhr von Flüssigkeiten im Auge, verfügt das chirurgische Gerät oder Handstück, mit welchem in das Auge eingegriffen wird, über eine Aspirationsleitung zur Absaugung und eine Infusionsleitung zum Ersatz der abgesaugten und/oder durch die Einführungsöffnung austretenden Leck-Flüssigkeit. Speziell muss dabei während der Operation der Augendruck (IOP = Inter Okular Pressure) zumindest annähernd konstant gehalten werden. Das Auge, bzw. ein Augenteil wie die Vorderkammer, soll während des Eingriffs weder zusammenfallen noch aufgebläht werden. Entsprechend erfolgt parallel zur Absaugung durch die Aspiration gleichzeitig eine Infusion, also Zuführung von Flüssigkeit.

Im Stand der Technik wird bei der Infusion vom Arzt meist ein gewünschter Infusionsdruck vorgegeben. Dies erfolgt dabei mit einem fixen oder motorisch höhenverstellbaren Ständer für die Infusionsflasche oder den Infusionsbeutel. Entsprechend des Höhenunterschieds zwischen Patienten und Flasche ergibt sich dabei ein hydrostatischer Druck, welcher Druck im Auge bei derartigen Eingriffen beispielsweise größenordnungsmäßig bei ca. 30 mbar über Atmosphärendruck liegen kann.

Eine derartige Infusion ist zwar einfach zu implementieren, hat aber durchaus Nachteile, beispielsweise ist die Einstellung eines gewünschten Infusionsdrucks eher statisch bzw. nur mit einer sehr geringen Dynamik veränderbar. Die Reaktion der Infusionsdruckänderung ist träge. So kann beispielsweise von Seiten der Infusion kaum oder gar nicht auf Unregelmäßigkeiten während einer Operation reagiert werden.

Es sind Systeme bekannt, bei welchen in den Infusionsbehälter Druckluft eingeblasen werden kann um den Infusionsdruck vorzugeben. Diese sind jedoch kompliziert in der Handhabung, sind Aufwändiger anzuschließen, bergen Gefahren eines Verlusts der Sterilität. Auch können Infusionsdruckveränderungen nur langsam umgesetzt werden. Für Druckverringerungen sind dabei speziell lange Kanülen notwendig, welche den Flüssigkeitsspiegel in der Flasche überragen. Bei dieser Lösung können auch nur spezielle Flaschen verwendet werden, welche geometrisch zur verwendeten Kanülenlänge passen.

Es ist eine Aufgabe der vorliegenden Erfindung, eine ophthalmologische Apparatur zu verbessern bzw. eine verbesserte Wechselkassette für eine derartige Apparatur bereitzustellen.

Eine Aufgabe ist dabei, speziell die Infusion in das Auge, welche z.B. im Rahmen einer Saugspülung während einer Phakoemulsifikation erfolgt, zu verbessern und zu vereinfachen.

Es ist insbesondere eine Aufgabe dabei eine einfache, sichere und zuverlässige Einhaltung eines gewünschten Augendruckes während eines Eingriffs sicherzustellen, speziell auch bei ungeplanten Vorkommnissen. Dabei soll auch die Bedienbarkeit und Handhabbarkeit des Geräts nicht verkompliziert, sondern potentiell vereinfacht werden. Auch gilt es, mögliche Fehlerquellen bei der Handhabung sowie bei der eigentlichen Verwendung der ophthalmologischen Apparatur und/oder Kassette zu reduzieren. Weitere Aufgabe können auch darin bestehen, nachteilige parasitäre Effekte im Stand der Technik, beispielsweise Druckabfall in den Schläuchen, Druckminderung durch Luftfilter in der Tropfkammer, etc. vermindern oder ausgleichen zu können.

Diese Aufgaben werden erfindungsgemäß durch die Merkmale der unabhängigen Ansprüche und/oder durch Merkmale der abhängigen Ansprüche gelöst bzw. diese Lösungen weitergebildet.

Die vorliegende Erfindung betrifft ein ophthalmologisches Gerät welches eine Saugspülung mit einer Aspirations-Funktion und einer Infusions-Funktion bereitstellt. Es weist einer Aspirations-Fördereinrichtung zur motorisch angetrieben Abführung von Flüssigkeit von einem chirurgischen Instrument in einen Abfallbehälter auf. Es wird also eine aktive Aspiration von Flüssigkeit mittels eines chirurgischen Eingriffwerkzeugs, welches nicht Teil dieser Erfindung ist, von einem Patienten hin zum Abfallbehälter bereitgestellt. Diese Fördereinrichtung kann insbesondere in Form einer Peristaltik-Pumpe oder einer Venturi-Aspiration mit Unterdruck-Absaugung ausgebildet sein.

Erfindungsgemäß weist das Gerät zudem eine Infusions-Fördereinrichtung zur motorisch angetriebenen Zuführung eines Infusionsmediums von einem Infusionsbehälter über einen Infusions-Anschluss und eine Leitung zum chirurgischen Instrument auf. Es wird also eine aktive Infusion mit einer forcierten Förderung des Infusionsmediums vom Infusionsmediums-Vorratsbehälter hin zu einem beim Patienten in Eingriff befindlichen chirurgischen Eingriffwerkzeugs bereitgestellt. Das Infusionsmedium kann dabei insbesondere eine Infusionsflüssigkeit sein, beispielsweise eine BSS. In der weiteren Beschreibung wird der einfachem Verständlichkeit wegen meist auf Infusionsflüssigkeit als Infusionsmedium verwiesen, was aber nicht zwingen einschränkend zu sehen ist. Alternativ kann in einer speziellen Ausführungsform auch ein Gas als Infusionsmedium dienen. Dabei kann neben einem Gasbehälter als Infusionsbehälter, speziell bei einer Nutzung von Luft als Infusionsmedium auch der OP als Infusionsbehälter fungieren.

Speziell weist das Gerät ein Steuerungsmodul auf, welches dazu ausgebildet ist ein bezüglich eines Infusionsdrucks und/oder eines Infusionsvolumens kontrollierte aktive Infusion bereitzustellen.

Die Infusions-Fördereinrichtung ist dabei erfindungsgemäss als Peristaltikpumpe zur aktiven Volumen-Förderung des Infusionsmediums mit einem bekannten Fördervolumen ausgebildet.

Dabei können speziell die mit Flüssigkeit in Kontakt tretende Bereiche der Infusions-Fördereinrichtung sowie der Aspirations-Fördereinrichtung in einer gemeinsamen Wechselkassette für das Gerät ausgebildet sein. Das Gerät kann hierbei jeweils einen zugehörigen Infusions-Antrieb für die Infusions-Fördereinrichtung und, insbesondere getrennt davon, einen zugehörigen Aspirations-Antrieb für die Aspirations-Fördereinrichtung aufweisen.

Die Aspirations- Fördereinrichtung und die Infusions-Fördereinrichtung können dabei in ihrer Ausgestaltung und Dimensionierung gleichartig - insbesondere gleich - aufgebaut sein. Speziell kann dies von Vorteil sein, da dabei eine gleichartige Ansteuerung der beiden Fördereinrichtungen auch eine gleichartige Volumenförderung bewirkt.

Zwischen Infusions-Fördereinrichtung und dem chirurgischen Instrument ist dabei eine Druckmesseinrichtung mit einem Druck- oder Kraftsensor im Gerät ausgebildet, mit welcher ein Infusionsdruck des Infusionsmediums im Gerät ermittelbar ist. Die Druckmesseinrichtung kann beispielsweise mit einer flexiblen Membrane in der Wechselkassette und einem zugehörigen Druck- oder Kraftsensor im Gerät ausgebildet sein, welche bei eingelegter Kassette eine Wirkverbindung eingehen, mittels welcher ein Druckwert des Infusionsmediums in der Kassette bestimmbar ist. Speziell kann dabei der ermittelte Infusionsdruck mit einer automatischen Ansteuerung der Infusions-Fördereinrichtung der ermittelte Infusionsdruck auf einen Sollwert ausregelbar sein - beispielsweise mittels einer hierfür ausgebildeten Reglereinheit im Gerät.

Zwischen Infusions-Fördereinrichtung und chirurgischen Instrument kann weiters eine Ripple-kompensation ausgebildet sein. Diese kann insbesondere als eine passive Ripple-kompensation in Form eines flexiblen Leitungsbereichs in der Wechselkassette ausgebildet sein, welcher mittels elastischer Verformung des flexiblen Leitungsbereichs einen Ausgleich von Schwankungen im Fördervolumen und/oder Förderdruck der Infusions-Fördereinrichtung bewirkt.

Das Ophthalmologisches Gerät kann zusätzlich mit einer motorisch angetriebene Höhenverstellung des Infusionsbehälters sowie mit einer Bypass-Ventileinrichtung zur optionalen Umgehung Infusions-Fördereinrichtung ausgebildet sein. Dabei kann die Bypass-Ventileinrichtung in der Wechselkassette passiv, z.B. als Flüssigkeitskanal-Quetschbereich, ausgebildet sein und von einem Ventilantrieb im Gerät mechanisch betätigt werden.

Entlang eines Pfades der Wechselkassette, in welchem die Infusionsflüssigkeit zugeführt wird, kann zudem ein Luftblasensensor ausgebildet sein. Dieser kann beispielsweise mit einem Sichtfenster in der Wechselkassette und einem optischen Überwachungssystem im Gerät ausgebildet sein.

Dieser kann eine Verfälschung eines Messwertes des Infusionsdrucks, eine Verringerung der Dynamik bei einer Infusionsdruckänderung durch elastische Luftblasen im Infusions-system und/oder eine Infusion von Luft ins Auge verhinderbar machen. Ferner kann beim Füllen des Schlauchsystems erkannt werden, wann die Wassersäule bei der Kassette ankommt, womit beispielsweise der Füllzyklus zeitlich abzukürzen werden kann. Auch kann mit diesem Sensor erkannt werden, wenn anstelle von BSS nur noch Luft aus der Infusionsflasche kommt. Eine Fehlermeldung kann dann das OP Personal warnen und dazu auffordern, eine neue BSS Flasche anzuschliessen bevor das Auge kollabiert.

Die Infusions-Fördereinrichtung kann dabei ausgebildet sein mit zumindest einem, zumindest annähernd kreisbogenförmig ausgebildeten Quetschkanal aus einem elastischen Material in der Wechselkassette, dessen Quetschkanal-Querschnitt von geräteseitigen Aktuatoren durch Rollen an zumindest einer Stelle abdichtend quetschbar ist und diese Stelle entlang des Quetschkanals (94) beweglich ist. Dabei kann insbesondere eine Quetschkanal-Querschnittsfläche entlang des ungequetschten Quetschkanals variierend ausgebildet sein. Speziell können auch optional mehrere Quetschkanäle von einer einzigen Infusionsfördereinrichtung hydraulisch parallel angeordnet und phasenversetzt angesteuert werden, um Ripple-Effekte der Peristaltikpumpe zu vermeiden.

Eine erfindungsgemäße Wechselkassette für ein ophthalmologisches Gerät kann damit mit zumindest einem rigiden Hartteil als Gehäuse der Wechselkassette sowie zumindest einem elastischen Weichteil ausgebildet sein. Das Hartteil kann insbesondere aus einem Hartkunststoff, speziell einem Thermoplast wie z.B. einem Polypropylen (PP) oder Polyethylen (PE) ausgebildet sein. Das Weichteil kann insbesondere aus einem Elastomer oder einem thermoplastischen Elastomer ausgebildet sein. Dabei kann zumindest eines der Hartteile mit zumindest einem Weichteil als ein einstückiges Mehrkomponenten-Spritzgussteil ausgebildet sein. Das Hartteil und das Weichteil bilden dabei interne Flüssigkeitskanäle der Wechselkassette aus, wobei das Weichteil zumindest teilweise von außen zugänglich ist. Dabei sind speziell auch Bereiche des Weichteils als kassettenseitige Peristaltikpumpen-Quetschkanäle zur Förderung von Infusionsflüssigkeit in zumindest einem der Flüssigkeitskanäle ausbildet.

Das Weichteil kann dabei verschiedenste Funktionselemente der Wechselkassette ausbilden. Beispielsweise zumindest eines von:
- einen Quetschpumpbereich für Luft oder Flüssigkeit in den Flüssigkeitskanälen für eine Volumenförderung in Form einer Peristaltikpumpe,
- einen Ventileinrichtungsbereich zur Veränderung eines Durchflussquerschnitts in zumindest einem der Flüssigkeitskanäle,
- einen Drucksensorbereich zur Bestimmung eines Luft- oder Flüssigkeitsdruckes in zumindest einem der Flüssigkeitskanäle, und/oder
- einen Ripple-Kompensationsbereich zum Ausgleich von Druck- und/oder Volumen-Schwankungen einer Flüssigkeitsförderung mittels eines elastischen Flüssigkeitskanalbereichs.

Die Wechselkassette kann zudem weiters zumindest mit:
- einem Flaschenanschluss zum Anschluss eines Behälters zur Bereitstellung von Infusionsflüssigkeit,
- einem Infusionsanschluss zur Zuführung der Infusionsflüssigkeit zum ophthalmo-chirurgischen Operationsinstrument
- einem Aspirationsanschluss (50) zur Verbindung von zumindest einem der Flüssigkeitskanäle mit einem ophthalmo-chirurgischen Operationsinstrument, und
- einem Abfall-Anschluss (55) zur Abfuhr von Flüssigkeit in ein Abfallgebinde ( 56), und/oder
- einem Aspirations-Ventil ausgebildet sein.

Die Erfindung betrifft ebenfalls ein Verfahren zur automatischen Infusionsdruckregelung in einem ophthalmologischen Gerät, welche speziell im Rahmen einer Saugspülung erfolgen kann. Dabei erfolgen eine aktive und speziell motorisch gesteuerte Aspirations-Volumen-Förderung einer Aspirationsflüssigkeit von einem Aspirationsanschluss hin zu einem Abfallbehälter, und eine aktive und speziell motorisch gesteuerte Infusions-Volumen-Förderung einer Infusionsflüssigkeit von einem Infusionsbehälter hin zu einem Infusionsanschluss. Dabei erfolgen die Infusions-Volumen-Förderung und die Aspirations-Volumen-Förderung jeweils mittels eines peristaltischen Pumpens. Anstelle der Infusionsflüssigkeit kann für spezielle Anwendungen in der Vitrektomie mit der erfindungsgemässen Infusions-Fördereinrichtung alternativ auch Luft anstellen von Flüssigkeit gefördert werden - also eine kontrollierte Infusion von Luft oder einem anderen Gas bereitgestellt werden. Dieses wir weiter unten im Detail beschrieben.

Dabei können eine Messung eines Infusionsdrucks der Infusionsflüssigkeit (oder alternativ in speziellen Fällen auch der Infusionsluft) sowie eine Regelung eines Förder-Volumens der Infusions-Volumen-Förderung anhand des Infusionsdrucks erfolgen. Speziell kann diese Regelung unter Berücksichtigung eines Druckabfalls in einer Leitung hin zum Auge erfolgen, welcher mit einer Drucküberhöhung bei der Infusions-Volumen-Förderung zumindest teilweise kompensiert wird, sodass der im Auge gewünschte Druck auch tatsächlich vorherrscht. Dieser Druckabfall kann erfindungsgemäß insbesondere unter Berücksichtigung einer Fliessgeschwindigkeit der Infusionsflüssigkeit bestimmt werden, da die Fliessgeschwindigkeit anhand eines aktuellen Förder-Volumens der Infusions-Volumen-Förderung ermittelbar ist.

Erfindungsgemäß kann ebenfalls ein Abstimmen des Förder-Volumens der aktiven Infusion zu einem Kennwert der aktiven Aspiration erfolgen. Speziell kann dabei eine Abhängigkeit einer Bewegung einer Infusions-Peristaltikpumpe von einer Bewegung einer Aspirations-Peristaltikpumpe implementiert sein. Dabei kann insbesondere ein Bestimmen eines Aspirationsunterdrucks bei der Aspirations-Volumen-Förderung erfolgen und bei einem Anstieg des Aspirations-unterdrucks ein temporäres Erhöhen des Infusionsdrucks erfolgen, beispielsweise im Falle von Aspirations-Okklusionen. Mit dieser temporären Erhöhung des Infusionsdruckes kann einer unerwünschten Druckminderung im Auge während dem Lösen der Aspirations-Okklusion entgegengewirkt werden.

Erfindungsgemäß erfolgt ein Verfahren zur Ansteuerung einer aktiven Infusion in einem ophthalmologischen Gerät zur Saugspülung durch eine Steuerungseinheit mit einem Ermitteln einer Aspirations-Fördermenge einer Aspiration der Saugspülung und einem Vorsteuern einer Infusions-Fördermenge der aktiven Infusion zum Ausgleich der Aspirations-Fördermenge. Dabei wird zusätzlich ein Bestimmen eines Infusionsdrucks der aktiven Infusion und ein Nachregeln der Vorgesteuerten Infusions-Fördermenge zur Erzielung eines gewünschten Infusionsdrucks durchgeführt.

Einige hier beschriebene Verfahren werden vorzugsweise zumindest teilweise als programmierte Abläufe bereitgestellt. Die Erfindung betrifft deshalb auch Computerprogrammprodukte mit Programmcode, der auf einem maschinenlesbaren Träger gespeichert ist, oder als ein Computer-Daten-Signal, verkörpert durch eine elektromagnetische Welle (z.B. etwa als Funksignal) bereitgestellt ist, zur Durchführung des obigen Verfahrens. Dabei kann der Programmcode speziell ein automatisches Durchführen von hier angeführten Steuerungs- und/oder Regelungs-Aufgaben, sowie von Ablaufsequenzen in einem ophthalmologischen Gerät ausführen bzw. hierzu ausgebildet sein.

Das erfindungsgemäße Verfahren und die erfindungsgemäße Vorrichtung werden nachfolgend anhand von in den Zeichnungen schematisch dargestellten, konkreten Ausführungsbeispielen rein beispielhaft näher beschrieben, wobei auch auf weitere Vorteile der Erfindung eingegangen wird. Im Einzelnen zeigen:
Fig. 1 ein erstes Block-Schema einer erfindungsgemäßen ersten Ausführungsform eines ophthalmologischen Geräts;
Fig. 2 ein zweites Block-Schema einer erfindungsgemäßen zweiten Ausführungsform eines ophthalmologischen Geräts;
Fig. 3a und Fig. 3b eine erste Ausführungsform eines Beispiels eines erfindungsgemäßen Wechselteils in Form einer austauschbaren Kassette für ein ophthalmologisches Gerät in 3D Ansichten von links und rechts;
Fig. 4 eine Darstellung einer beispielhaften erfindungsgemäßen Ausführungsform eines Wechselteils, welches in eine entsprechendes Beispiel eines erfindungsgemäßes ophthalmologisches Geräts eingelegt ist;
Fig. 5 eine Aufsichtdarstellung einer erfindungsgemäßen Ausführungsform einer Infusions-flüssigkeitsförderung;
Fig. 6a und Fig. 6b Ausführungsformen von Beispielen eines erfindungsgemäßen Wechselteils beim Einlegen in das Gerät in einer Schnittdarstellung;
Fig. 7a und Fig. 7b Ausführungsformen eines Beispiels eines erfindungsgemäßen Wechselteils mit einer Ausführungsform einer optionalen Ripple-Reduktion;
Fig. 8a und Fig. 8b eine Darstellung einer beispielhaften erfindungsgemäßen Ausführungsform eines Wechselteils mit erfindungsgemäßer aktiver Infusionsfördereinrichtung;
Fig. 9 ein erstes Beispiel einer Ausführungsform einer erfindungsgemäßen aktiven Infusion in Anwendung;
Fig. 10 ein zweites Beispiel einer Ausführungsform einer erfindungsgemäßen aktiven Infusion in Anwendung.

Die Darstellungen in den Figuren dienen lediglich zur Illustration und sind, sofern nicht explizit angegeben, nicht als maßstäblich zu betrachten. Gleiche oder funktionell ähnliche Merkmale sind, insoweit praktikabel, durchgängig mit denselben Bezugszeichen versehen und werden gegebenenfalls mit einem Buchstaben als Index unterschieden. Die dargestellten Schemata zeigen jeweils die technische Grundstruktur, welche von einem Fachmann entsprechend allgemeinen Grundsätzen ergänzt oder modifiziert werden können.

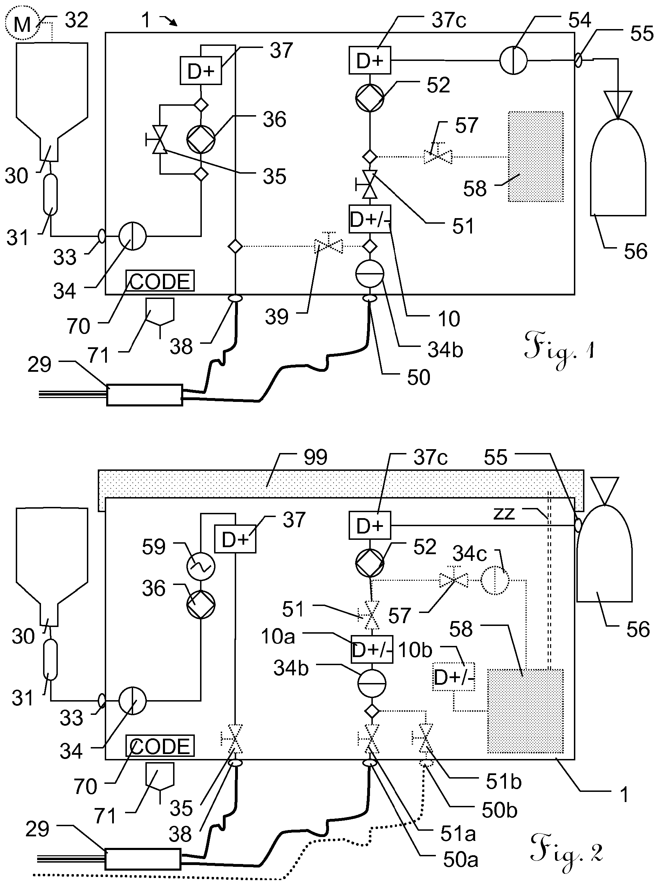

**Fig. 1** zeigt ein mögliches Beispiel eines Blockdiagramms einer Ausführungsform einer erfindungsgemäßen ophthalmologischen Kassette 1 mit zumindest einer erfindungsgemäßen aktiven Infusion. Es kann sich in diesem Beispiel speziell um eine Infusionseinrichtung in einer austauschbaren Kassette 1 eines ophthalmologischen Geräts 99, mit welchem eine Saugspülung durchführbar ist handeln. In der linken Hälfte der Figur ist eine aktive Infusionseinrichtung gezeigt, in der rechten Hälfte eine Aspirationseinrichtung.

Ein Behälter 30, also beispielsweise eine Infusionsflasche oder ein Infusionsbeutel, stellt dabei eine Infusionsflüssigkeit bereit, z.B. eine BSS (Balanced Salt Solution) oder eine andere Infusionsflüssigkeit. Diese Infusionsflüssigkeitszufuhr kann z.B. mit einer Einrichtung zur Kontrolle des Füllstandes ausgestattet sein. Hierzu kann beispielsweise der Behälter 30, eine Tropfkammer 31, ein Anschlussschlauch und oder ein kassetteninterner Flüssigkeitskanal mit einem verkabelten oder kabellosen Sensor auf Entleerung überwacht werden. Der Anschluss 33 kann dabei - in eingelegtem Zustand der Kassette 1 von außen zugänglich - an der Kassette 1 angeordnet sein, und beispielsweise über einen Schlauch mit dem Infusionsbehälter 30, respektive mit der dazwischenliegenden Tropfkammer, verbunden werden.

Wie im Stand der Technik bekannt, kann optional auch durch eine, vorzugsweise motorisiert und automatisch erfolgende, Höhenverstellung der Aufhängung des Behälters 30, welche hier durch den Antrieb 32 symbolisiert wird, der Infusionsflüssigkeitsdruck am Flaschen-Anschluss 33 der Kassette 1, (bzw. im gesamten Infusionssystem) durch Variation der Höhe der Behälteraufhängung verändert werden. Mit der erfindungsgemäßen aktiven Infusion ist dieses Höhenverstellen aber nicht zwingend erforderlich, kann aber als umschaltbare Option und/oder als zusätzliche Sicherheit oder Redundanz zusätzlich am erfindungsgemäßen Gerät ausgebildet sein.

Bei einer anderen Ausführungsform einer erfindungsgemäßen Kassette 1 kann erfindungsgemäß eine aktive Infusion ausschließlich mittels einer aktiven Flüssigkeits-Fördereinrichtung 36 erfolgen. Hierzu ist eine Pumpeinrichtung, wie beispielsweise etwa eine Peristaltikpumpe oder eine andere Fördereinrichtung für Flüssigkeit im Infusionssystem ausgebildet. Bei einer derartigen Ausführungsform kann optional auch auf eine Infusions-Ventil 35 und/oder eine Höhenverstellung des Infusionsbehälters 30 verzichtet werden. Durch entsprechende Ansteuerung der Fördereinrichtung 36 kann dabei der Infusionsdruck und/oder das Fördervolumen der Infusion variiert werden, insbesondere gesteuert oder geregelt werden.

Eine weitere, bevorzugte Ausführungsform einer erfindungsgemäßen Kassette 1 kann auch - wie in der Figur gezeigt - beides, eine Infusionsbehälter-Höhenverstellung 32 und eine Infusionsflüssigkeits-Fördereinrichtung 36 aufweisen. Die erfindungsgemäße Kassette 1 kann in einer derartigen Ausführungsform zusätzlich mit einer hier beschriebenen Einstellung oder Regelung des Infusionsdrucks über eine Veränderung des Höhenunterschieds zwischen dem Behälter 30 der Infusionsstelle erfolgen. Dabei kann insbesondere ein Ventil 35 als Bypass zur Fördereinrichtung 36 ausgebildet sein. Somit kann mit derselben Kassette 1 entweder eine aktive (also durch die Fördereinrichtung 36 aktiv initiierte) oder eine hydrostatische (also durch einen Höhenunterschied bewirkte) Infusionsdruck-Variation bereitgestellt werden. Insbesondere kann dabei bei Bedarf zwischen diesen beiden Prinzipien nahtlos - also z.B. auch während des Betriebs - umgeschaltet werden, beispielsweise entsprechend der aktuellen Anforderungen der ausgeführten Operation oder entsprechend den Vorlieben des Chirurgen. Auch spezielle Anwendungen, bei welchen gleichzeitig zwei unterschiedliche und unabhängige Infusionsdrücke benötigt werden, sind mit einer derartigen erfindungsgemässen Ausführungsform durchführbar, indem eine erste druckregulierbaren Infusion mittels der hydrostatischen Höhenverstellung über eine erste Leitung hin zum Auge sowie eine zweite druckregulierbare Infusion mit der aktiven Infusion mittels der Infusions-Fördereinrichtung in der Kassette über eine zweite Leitung hin zum Auge angewandt wird.

Das erfindungsgemäße Infusionssystem weist zudem eine Infusions-Druckmesseinrichtung 37 auf, mittels welcher ein Druck im Infusionssystem ermittelbar, überwachbar und/oder regelbar ist. Vorzugweise ist diese Infusions-Druckmesseinrichtung 37 nach dem Infusions-Ventil 35 und/oder der Pumpeinrichtung 36 angeordnet, sodass deren ermittelter Druck jenem entspricht, welcher dem Auge zugeführt wird. Da im Infusionssystem gegenüber der Atmosphäre nur Überdruck herrschen kann (oder zumindest sollte), kann diese Infusions-Druckmesseinrichtung 37 derart ausgebildet sein, dass mit dieser nur Überdruck bestimmbar ist, und nicht notwendigerweise auch Unterdruck (obgleich eine derartige Variante ebenfalls anwendbar wäre). Eines von vielen möglichen Beispielen einer konkreten Ausführungsform kann etwa mit einem zu einer Druckkammer ausgeweiteten Teil des Infusions-Flüssigkeitskanals gebildet werden, welche nach außen hin eine flexible Membrane aufweist. Bei eingelegter Kassette 1 liegt diese Membrane an einer Sensitivitätsfläche eines Kraft- oder Drucksensor im Gerät 99 an. Die durch einen Überdruck im Flüssigkeitskanal gegenüber der Atmosphäre auf die Membrane ausgeübte Kraftwirkung kann damit als Messwert für den Überdruck im Flüssigkeitskanal bestimmt werden.

Die Infusionsflüssigkeit wird mittels der erfindungsgemäßen Kassette 1 mit erfindungsgemäßer aktiver Infusion mit einstellbarem Druck und/oder Fördervolumen an einem externen Infusions-Anschluss 38 der Kassette 1 bereitgestellt, welcher mit einer Leitung oder einem Schlauch hin zum chirurgischen Eingriffswerkzeug, also z.B. dem Operations-Handstück 29, verbunden werden kann, sodass die Infusion ins Auge mit einstellbarem und/oder regelbarem Druck und/oder Volumen bereitgestellt werden kann. Um Sicherheit zu gewährleisten, kann dabei der tatsächliche vorherrschende Druck der Infusion hin zum Patienten mit einem entsprechenden Drucksensor 37 überwacht werden, gegebenenfalls können dabei auch Druckverluste im Leitungssystem zum Patienten Berücksichtigung finden - speziell auch dynamik-bedingte Druckverluste, da ja erfindungsgemäß durch die Fördereinrichtung 36 das Fördervolumen und somit auch die Fliessgeschwindigkeit der Infusion bekannt ist.

Vorzugsweise ist nach einem Teilaspekt der Erfindung, die Anordnung und Ausbildung der Flüssigkeitskanäle innerhalb der Kassette 1 derart, dass bei einem Füllen der Flüssigkeitskanäle mit Flüssigkeit, dieses Füllen stets (zumindest substantiell) von unten nach oben erfolgt - womit allfällige Luftblasen (dem Mediumsdichtenverhältnis entsprechend) nach oben verdrängt und vom Patienten weg abgeführt werden und eine Ansammeln von Luftblasen vermieden wird. Ein derartiges Füllen der Flüssigkeitskanäle der Kassette 1 erfolgt dabei insbesondere nach dem Einlegen der Kassette 1 bei deren Inbetriebnahme. Dabei kann z.B. entsprechend dieses Aspekts der Erfindung, die Anordnung der Anschlüsse von unten nach oben in der Reihenfolge Abfallbeutel 55 - Aspiration-A vom Patienten 12a,50 - ev. Aspiration-B 12b - Infusion zum Patienten 38 - Infusionsflasche 33 ausgebildet sein.

Die erfindungsgemäße Kassette 1 kann dabei zumindest einen Luftblasensensor 34 im Infusionssystem aufweisen, mit welchem eine korrekte Funktion überwacht und eine Infusion von Luft vermieden werden kann. Der Luftblasendetektor 34 kann dabei insbesondere optisch - beispielsweise aufgrund unterschiedlicher Lichtbrechungseigenschaften von Luft und Flüssigkeit, oder auch auf anderen Prinzipen arbeiten, mit welchen zwischen einem Vorhandensein von Gas oder Flüssigkeit im Infusionssystem unterschieden werden kann. Beispielsweise kann das Prinzip unterschiedlicher Winkel der Totalreflexion von Flüssigkeit im Vergleich zu Luft, unterschiedliche Lichtleiteigenschaften von Flüssigkeit und Luft, oder eine anderes Wirkprinzip genutzt werden, beispielsweise ein kapazitives. Ein Beispiel einer konkreten Ausführungsform kann mit einem, zumindest in der genutzten Wellenlänge optisch transparenten Sichtfenster in den Flüssigkeitskanal ausgebildet sein, welches in einer Außenschale 41,42 der Kassette 1 implementiert sein kann. In einer Ausführungsform kann dabei beispielsweise die Außenschale aus transparentem Material ausgebildet sein.

Hier gezeigt ist auch ein optionaler erfindungsgemäßer Pfad vom Infusionssystem hin zum Aspirationssystem der Kassette 1, welcher mit einem Venting-Ventil 39 freigebbar ist. Mit diesem kann beispielsweise, insbesondere bei Inbetriebnahme der Kassette 1, eine Befüllung des Aspirationssystems mit Flüssigkeit erfolgen, und/oder es kann eine Rückspülung von Infusionsflüssigkeit durch das Aspirationssystem durchgeführt werden, beispielsweise zum Lösen von Okklusionen (Reflux) oder generell zum Unterdruckabbau im Aspirationspfad, z.B. wenn der Arzt via Fußschalter den gewünschten Unterdruck abbauen möchte. Für diese Funktionalitäten können allenfalls noch weitere Ventile zur entsprechenden Schaltung der Flüssigkeitsströme in der Kassette vorhanden sein.

Das Aspirationssystem der erfindungsgemäßen Kassette 1 wird über einen Aspirationsanschluss 50 mit einer Leitung oder einem Schlauch ebenfalls hin zum chirurgischen Eingriffswerkzeug 29 verbunden. Auch hier kann wiederum zumindest ein Luftblasensensor 34b im Aspirationssystem vorhanden sein. In der Figur gezeigt ist auch ein Aspirations-ventil 51. Auch kann im Aspirationssystem wiederum eine Druckmessung mit einer Druckmesseinrichtung 10 erfolgen, welche jedoch sowohl Unter- als auch Überdrücke gegenüber der Atmosphäre erfassen kann. Um entsprechend Kräfte in positiver als auch in negativer Richtung erfassen zu können, muss daher eine Anbindung der Druckmessmembrane der Kassette an die zugehörige Druckmess-Sensorik im Gerät zur Übertragung von positiven als auch negativen Werten ausgebildet sein. Beispiele solcher bekannten Druckerfassungseinrichtungen finden sich etwa der am selben Tag vom selben Anmelder eingereichten Patentanmeldung EP 16197018, welche hiermit per Referenz mit einbezogen wird, oder allenfalls auch den darin zitierten Referenzen.

Zur Erzielung der Aspirationswirkung zeigt die Figur zwei Varianten. Zum einen eine Peristaltik-Aspiration und zum anderen eine Venturi-Aspiration. Eine erfindungsgemäße Ausführungsform einer Kassette 1 kann entweder nur die Peristaltik-Aspiration aufweisen, oder eine andere erfindungsgemäße Ausführungsform einer Kassette 1 kann nur die Venturi-Aspiration aufweisen oder eine weitere erfindungsgemäße Ausführungsform kann sowohl die Peristaltik-Aspiration als auch die Venturi-Aspiration aufweisen.

Bei der Venturi-Aspiration wird die Aspirationswirkung durch einen Luft-Unterdruck bewirkt, welcher meist mit einer namensgebenden Venturi-Düse oder einer Vakuumpumpe erzeugt wird. Diese Varianten sind hier durch das Venturi-Ventil 57 und das Unterdrucksystem 58 symbolisiert. Speziell kann auch ein so genanntes "Clean Venturi"-System angewandt werden, welches in der am selben Tag vom selben Anmelder eingereichten internationalen Anmeldung EP 16196998 beschrieben ist und hiermit per Referenz miteinbezogen ist. Bei einer Venturi-Aspiration kann insbesondere ein gemessener Wert des Aspirations-Unterdrucks als Basis zur Ansteuerung der aktiven Infusions-Fördereinrichtung 36 genutzt werden, z.B. wie dies weiter unten beschrieben ist. Speziell kann die erfindungsgemässe aktive Infusions-Fördereinrichtung 36 auf Basis von aktuell vorherrschendem Infusionsdruck und aktuell vorherrschendem Aspirationsunterdruck durch eine hierfür vorgesehene Kontrolleinheit gesteuert und/oder geregelt werden. Optional oder Alternativ können dabei von der Kontrolleinheit auch Werte einer aktuellen Fördermenge der Infusion und/oder Aspiration mit einbezogen werden.

Bei der Peristaltik-Aspiration wird eine FlüssigkeitsFördereinrichtung 52 zur Erzeugung der Aspirationswirkung angewandt. Die Fördereinrichtung 52 kann insbesondere eine Peristaltikpumpe sein, optional ist aber auch z.B. eine Membranpumpe oder eine andere Flüssigkeitsfördereinrichtungen einsetzbar. Die aspirierte Flüssigkeit wird dabei vorzugsweise in einen entsprechenden AbfallBehälter 56 gefördert, welcher vorzugsweise an einem entsprechenden Abfall-Anschluss 55 außerhalb der Kassette 1 angeschlossen ist. Dabei kann wiederum ein Drucksensor 37c, insbesondere für Überdruck, genutzt werden, um einen Druckanstieg bei vollem Abfallbeutel zu detektieren.

Die erfindungsgemäße Kassette 1 kann auch eine, insbesondere mechanische und/oder optische, Codierung 70 aufweisen, welche von einer entsprechenden Leseeinheit 71 des Geräts 99 erfasst und ausgewertet werden kann. Anhand dieser Codierung kann - beispielsweise bei Einwegkassetten - ein mehrfache oder nichtsterile Nutzung verhindert werden, eine spezifische Ausführungsform der eingelegten Kassette und deren optionalen Funktionalitäten, etc. vom Gerät 99 erkannt werden.

Bei speziellen Vitrektomie-Eingriffen wird beim Entfernen de Glaskörpers zuerst ein Gas, meist mit einem Bakterienfilter gereinigte Raumluft aus dem OP, in das Auge gepumpt, meist in etwa mit einem Überdruck von ca. 20 bis 120 mmHg. Infolge wird diese eingepumpte Luft dann durch ein Silikonöl ersetzt, welches die abgelöste Netzhaut wieder an die Aderhaut anpresst, sodass diese wieder anwachsen kann. Im Stand der Technik wird hierzu eine speziell für diesen Zweck vorgesehen Luftpumpe im Gerät benötigt, welche jedoch meist nur sehr selten tatsächlich zum Einsatz kommt. Wird als erfindungsgemässe aktive Fördereinrichtung 36 für die Infusion speziell eine Peristaltikpumpe eingesetzt, kann diese anstelle von Infusionsflüssigkeit auch zur Förderung von Gasen eingesetzt werden. Dies ergibt nicht nur einen einfacheren Geräteaufbau durch einen Verzicht auf die spezielle Luftpumpeinrichtung, sondern hat vor allem auch Vorteile bezüglich der Hygiene und Sterilität, da die Wechselkassette 1 sterilisiert ist und auch regelmässig ausgetauscht wird. Als optionales Zubehör kann hierbei beispielsweise ein vorzugsweise ebenfalls steriler und/oder einmalverwendeter Bakterienfilter am Flaschenanschluss der Wechselkassette 1 angebracht werden, welcher die in diesem Falle angesaugte Infusions-Luft filtert.

**Fig. 2** zeigt eine weitere beispielhafte Ausführungsform einer erfindungsgemäßen Kassette 1 in einem Blockdiagramm. Die zu Fig. 1 funktional gleichen oder gleichwertigen Merkmale sind dabei mit denselben Bezugszeichen versehen und es wird auf deren obenstehende Beschreibung verwiesen.

Die wesentlichen Unterschiede dieser erfindungsgemäßen Ausführungsform zu Fig. 1 sind erfindungsgemäß, dass im Infusionsbereich lediglich eine aktive Infusion mittels einer Fördereinrichtung 36 erfolgt. Das Infusionsventil 35 ist hier nur optional als zusätzliche Absperrung gegenüber dem Patienten ausgebildet - kann aber auch weggelassen werden, insbesondere wenn die Flüssigkeitsfördereinrichtung 36 bereits abdichtend wirkt. Im Infusionspfad ist zusätzlich ein passives oder aktives Ripple-Kompensationselement 59 für eine Peristaltikpumpe 36 gezeigt. Eine passive Kompensation kann z.B. mit einem flexiblen Bereich des Flüssigkeitskanals ausgebildet werden, welcher die Wirkung eines Ausgleich-Balgs hat, welcher Druck- bzw. Fördermengen-Ripple abfedert, indem er sich entsprechend der Ripple verformt. Alternativ oder zusätzlich kann auch eine aktive Ripple-Kompensation angewandt werden, bei welcher ein geräteseitiger Aktor einen flexiblen Bereich eines Infusionsflüssigkeitskanals verformt und dabei Druck- bzw. Fördermengen-Ripple Effekte ausgleicht. Die Ansteuerung des Aktors kann dabei z.B. anhand von Werten des Drucksensors 37 und/oder anhand von Informationen über die Drehlage der Peristaltikpumpe 36 erfolgen. Erfindungsgemäss kann auch eine dynamische Variation der Drehgeschwindigkeit der Peristaltikpumpe derart erfolgen, dass der Ripple Effekt der Fördereinrichtung minimiert wird. Im Gegensatz zu einer gewöhnlichen, gleichförmigen Drehbewegung (mit allenfalls einer beschleunigten oder verzögerten Phase bei Fördermengen-Änderungen) wie dieses im Stand der Technik angewandt wird, erfolg hierbei keine strikt gleichförmige Drehbewegung des Rollenkopfes während der Förderung sondern die Drehgeschwindigkeit wird mit der Periodendauer der Rolleneingriffe derart moduliert, dass die mit dieser Periodendauer auftretenden Fördermengen-Ripple möglichst ausgeglichen werden. Es erfolgt also eine temporäre Beschleunigung oder Verzögerung der Rollenkopfbewegung, wenn eine Rolle in den Bereich des Einlasses oder Auslasses des Quetschkanals gelangt (Ein- oder Auslass, je nach dem auf welcher Seite der Ripple kompensiert werden soll, also bei Zufuhr oder Absaugung).

Im Aspirationsbereich ist erfindungsgemäß der Abfallbeutel 56 direkt an der Kassette 1 anbringbar. Auch ist neben dem Aspirationsanschluss 50a ein zweiter, optionale Aspirationsanschluss 50b mit entsprechenden Aspirationsventilen 51b und 50b zu deren Auswahl gezeigt. Der optionale Drucksensor 10b kann bei einem Venturi-System zusätzlich den Druck in dessen Saugkammer überwachen.

Erfindungsgemäss kann auch eine deutlich raschere Anpassung des Infusionsdruckes durchgeführt werden, als dies im Stand der Technik möglich ist. Dies kann insbesondere abhängig vom Operationsschritt oder von speziellen Gegebenheiten, die während der Operation auftreten, erfolgen.

Mit den erfindungsgemäss bekannten Daten bezüglich der Durchflussmenge der aktiven Infusion sowie der Aspiration kann auch auf die Dichtigkeit der Inzisionen (=Öffnung im Auge) erfolgen und diese Information kann zur Anzeige oder weiteren Verarbeitung bereitgestellt werden.

Die hier beschriebenen Ausführungsformen einer erfindungsgemässen aktiven Infusion bringen eine Vielzahl von Vorteilen gegenüber einer bekannten Infusionssystemen wie z.B. einer Schwekraft-Infusion oder einer Infusion mittels Einpumpen von Luft in die Infusionsflache. Speziell bietet eine erfindungsgemässe aktive Infusion, insbesondere mit einer Peristaltikpumpe, Vorteile wie etwa:
- Vereinfachung des Einrichtens der Operation und der Inbetriebnahme des Geräts - und somit Zeitgewinn, insbesondere da ein initales Befüllen des Infusionssystems sich deutlich einfacher und schneller gestaltet.
- Geringere Risiken bezüglich Sterilitätsfehlern, insbesondere beim Einrichten des Geräts.
- Schnellere Druckveränderungen während des Eingriffs als im Stand der Technik können durchgeführt werden, was viele bis dahin nicht ofenkundige Einsatzmöglichkeiten bietet.
- Unabhängigkeit von einer speziellen Bereitstellung der Infusionsflüssigkeit, insbesondere bezüglich Flaschengrösse und Flaschenart, da dies auf die erfindungsgemässe aktive Infusion keine Auswirkungen hat.
- Zusätzliche Überwachungsfunktionalitäten, Parameterauswertungen, Sicherheits- und Komfortfunktionen, etc. sind implementierbar.
- Es fällt weniger Abfall an, da Luftfilter, lange Nadeln für das Infusionsset, Schläuche, Verpackung etc. eingespart werden können. Lediglich die ohnehin für die Aspiration anfallende Wechselkassette wird benötigt - welche optional auch mehrfach sterilisierbar ausgeführt sein kann. Auch die Verbrauchsartikel-Kosten sinken.

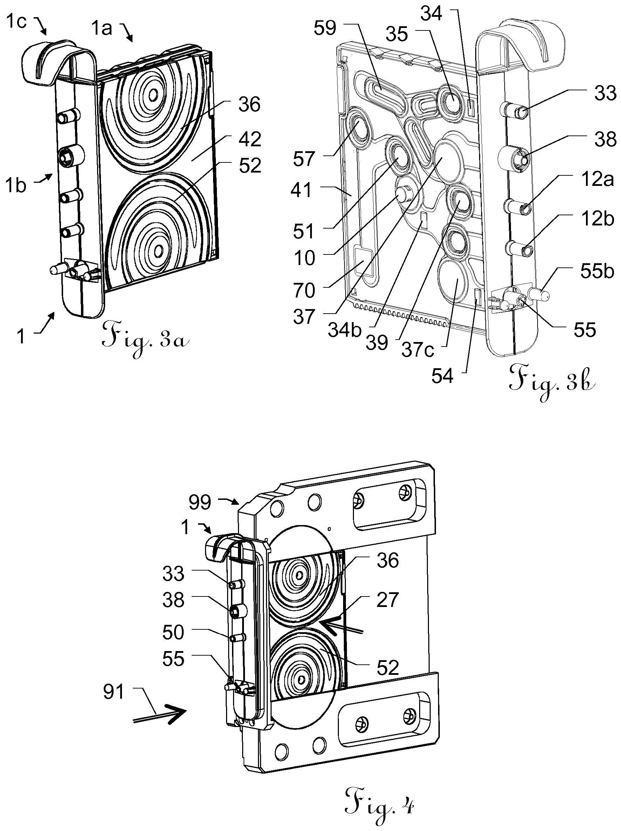

In **Fig. 3a** und **Fig. 3b** ist eine weitere Ausführungsform einer erfindungsgemäßen Kassette 1 jeweils in einer Ansicht von rechts und links vorne dargestellt. In den Figuren sind auch nochmals die zuvor beim Blockschema schon beschriebenen Funktionselemente gezeigt und gekennzeichnet. Die Kassette 1 ist in diesem Beispiel als eine, in ihren Außenkonturen zumindest annähernd quaderförmiger Basis-Kassette 1a mit einer überragenden Platte als Frontpartie 1b gezeigt. An der Frontpartie 1b sind Anschlüsse für Schlauchsysteme zu finden, insbesondere jene an das Operations-Handstücks, an die Infusionsflüssigkeits-Reservoir 30, und an einen Abfallbeutel 56. Vorzugsweise sind diese Anschlüsse 33,38,50,55 wie gezeigt mechanisch und/oder geometrisch codiert, um die Gefahr eines fälschlichen Anschließens und Verwechslungen auszuschließen.

In dem dargestellten Beispiel einer Ausführungsform sind die funktionalen Elemente der Kassette 1 jeweils auf beide der gezeigten Außenschalenhälften 41 und 42 der Kassette 1 aufgeteilt, womit ein kompakter Aufbau erzielbar ist. Dabei sind speziell auf einer Kassettenseite 42 die Quetschpumpen-Abschnitte 36 und 52, und auf der gegenüberliegenden Kassettenseite 41 die Ventile und Drucksensoren angeordnet. Eine derartige Aufteilung der funktionalen Elemente ist nicht zwingend, kann aber bezüglich einer Einspannung oder Klemmung der Kassette 1 im Gerät 99 vorteilhaft sein, bei welcher mit dem Anpressen des oder der Rollenköpfe der Peristaltikpumpe zugleich eine Klemmung, Fixierung und/oder Fein-Positionierung der Kassette 1 im Gerät 99 erfolgen kann. Dabei kann die zur Klemmung der Kassette im Gerät 99 erforderliche Bewegung nur von einer Seite, vorzugsweise von der Seite der Peristaltik-Rollen erfolgen. Die folgenden Figuren illustrieren weitere beispielhafte Ausführungsform einer erfindungsgemäßen Peristaltikpumpe einer erfindungsgemäßen Kassette 1 im Detail.

**Fig. 4** zeigt eine beispielhafte Ausführungsform einer erfindungsgemäßen Kassette 1 in Ihrer Endstellung in einem Kassettenschacht eines Geräts 99. Das Einlegen erfolgte durch ein einschieben der Kassette 1 in den Kassettenschacht in Einlegerichtung 91 und kann manuell oder auch zumindest teilweise automatisiert bzw. motorisiert erfolgen. Es ist dabei ein Teil des Kassettenschachts im Schnitt dargestellt, die Kassette 1 jedoch ungeschnitten. In der gezeigten Position der Kassette 1 im Gerät 99 kann ein andrücken von Rollern auf die Quetschbereiche der Peristaltikpumpen 36 und/oder 52 im wesentliche orthogonal zur gezeigten Seite Kassette 1 in Richtung 27 erfolgen, womit die Kassette 1 erfindungsgemäß zugleich im Gerät 99 positioniert und fixiert werden kann. In diesem eingelegten Zustand sind die geräteseitigen Sensoren und Aktoren bezüglich der Kassette in einer Lage, in welcher diese mit ihren jeweils zugehörigen Funktionselementen der Kassette 1 zweckbestimmt zusammenwirken können.

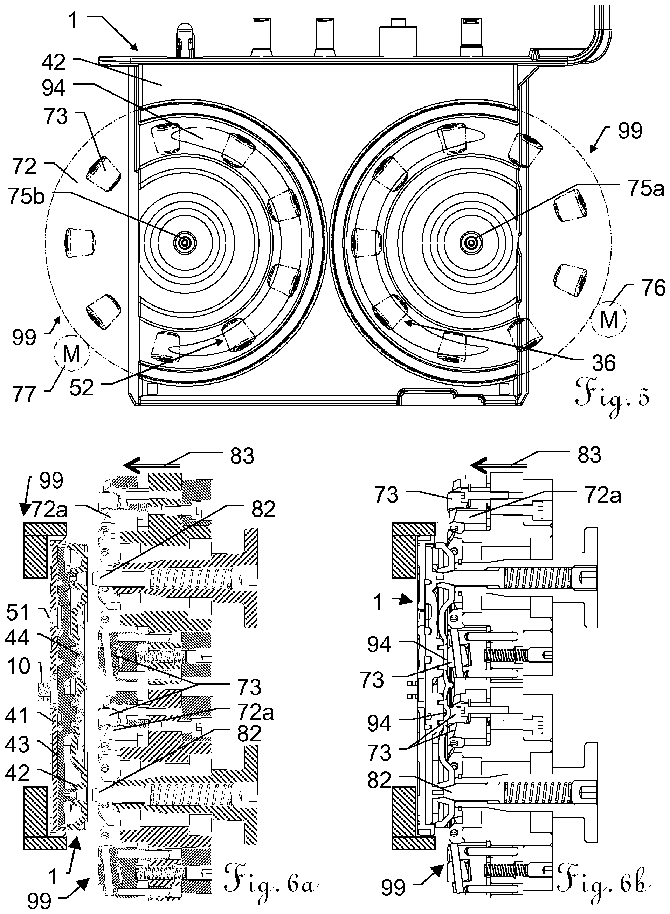

**Fig. 5** zeigt schematisch ein solches Klemmen und fixieren der eingeschobenen Kassette 1 im Gerät 99 in einer Seitenriss-Darstellung, mit Blickrichtung auf Peristaltikpumpen der Kassette 1 (Richtung 27 in Fig. 4). Dabei ist ein geräteseitiger Rollenkopf 72 mit Rollen 73 gezeigt, welche Rollen 73 auf dem wulstförmig aufgewölbten Quetschbereich 94 der Außenschale 42 abrollen, welcher aus elastischem Weichkunststoff 44 gebildet ist. Bei diesem Abrollen wird der Quetschbereich 94 von den Rollen 73 abdichtend auf das Kernteil 43 der Kassette 1 gedrückt und bei einer Drehung des Rollenkopfes 72 um das Drehzentrum 75 eine Förderung eines Volumens im Quetschbereich 94 zwischen den Rollen 73 bewirkt. Für die Drehung der Rollenköpfe 72 weist das Gerät 99 jeweils einen Infusions-Antrieb 76 für die Infusions-Fördereinrichtung 36 und einen Aspirations-Antrieb 77 für die Aspirations-Fördereinrichtung 52 auf.

In **Fig. 6a** ist die Kassette 1 in Einlegerichtung 91, welche in dieser Figur orthogonal zur Zeichnungsebene verläuft, in das Gerät 99 bis zu ihrer Endlage eingelegt, aber noch nicht geklemmt. Es handelt sich um eine Schnittdarstellung durch das rechte und linke Drehzentrum 75a und 75b der zwei getrennten Peristaltikpumpen 36 und 52 aus Fig. 5. Im gezeigten Beispiel ist die im Schnitt dargestellte Kassette 1 im wesentlichen dreiteilig aufgebaut, mit einer linken Außenschale 41, einer rechten Außenschale 42 und einem Kernteil 43. Vorzugsweise sind die Außenschalen 41 und 42 gegeneinander und/oder gegen das Kernteil 43 verschnappbar, verpressbar oder verquetschbar ausgebildet, also beispielsweise mit einem Hakensystem, einem Press-Fit System oder ähnlichem, welche insbesondere nicht wieder lösbar ausgestaltet sein können. Dabei können die Außenschalen 41 und 42 insbesondere als 2K-Spritzgussteile aus einem rigiden Hartkunststoff-Anteil und einem elastischer Weichkunststoff-Anteil ausgebildet sein. Der elastische Weichkunststoff-Anteil wird dann mit dem Kernteil 43 verspannt und gequetscht und/oder formschlüssig im Eingriff gebracht, sodass dabei die Flüssigkeitskanäle bzw. Leitungen innerhalb der Kassette 1 gebildet und abgedichtet werden. Im gezeigten Schnitt bildet der elastische Anteil 44 entlang der Flüssigkeits-kanäle Wülste aus, welche in zusammengebautem Zustand in Vertiefung des Kernteils 43 eingreifen - womit eine Flüssigkeitsabdichtung bewirkt wird, insbesondere durch Quetschung des Weichkunststoffs 44 und/oder Ausbildung einer Mäanderdichtung mit zumindest einer Stufung. Teilbereiche des Weichkunststoffs-Anteils 44 sind dabei in montiertem Zustand der Kassette 1 von außen zugänglich und bilden die Funktionselemente aus. Der Hartkunststoff-Anteil der 2K-Spritzguss-Außenschalen 41 und 42 weist dafür entsprechende Ausschnitte auf welche Zugang zum Weichkunststoff 44 schaffen. Eine allfällige Sterilisierbarkeit der gesamten Kassette, beispielsweise im Autokalven, etc. kann bei der Materialwahl der Kassettenmaterialen ebenfalls Berücksichtigung finden, falls eine mehrfach verwendbare Ausführungsform angestrebt wird.

In diesem Beispiel einer erfindungsgemäßen Ausführungsform wird nach dem Einlegen ein Klemmen der Kassette 1 gemeinsam mit einem Andrücken der Rollenköpfe 72 der Peristaltikpumpe in Richtung 83 bewirkt. Wie gezeigt kann dabei die Klemmkraft der Rollen 73 und/oder der Positionsausrichtung 82 mittels Federn bestimmt werden.

Ebenfalls erfindungsgemäß im Gerät 99 angeordnet sind die hier nicht einsehbaren Aktoren, welche auf die Ventile 35, 39,51,51a,51b und/oder 57 einwirken und beispielsweise als elektromagnetisch oder elektromotorisch oder pneumatisch betätigte Stößeln im Gerät ausgebildet sein, welche mechanisch auf die Ventilausformungen der Kassette 1 einwirken. Auch die Druckerfassungseinrichtung 10, speziell deren Kupplungselement, ist im eingelegten Zustand der Kassette 1 mit dem hier nicht gezeigten, geräteseitigen Kraftsensor 11 gekoppelt. Ebenso wie die kassettenseitige Membrane des Drucksensors 37 und/oder 37c mit einem geräteseitigen Kraft- oder Drucksensor in eine funktionsgemäße Wirkverbindung gebracht ist.

**Fig. 6b** zeigt die Kassette 1 in geklemmtem Zustand im Gerät 99, in welchem alle Sensoren und Aktoren des Geräts 99 mit ihren Gegenstücken der Kassette 1 zur Interaktion korrespondieren. Im gezeigten Beispiel erfolgt dieses durch ein Klemmen der Kassette 1 im Gerät 99, welches durch andrücken der Rollenköpfe 72 an die Kassette in Richtung 83 erfolgt. Somit wird die Kassette 1 in der Zeichnungsebene nach links auf entsprechende mechanische Anschläge gedrückt. Dabei Quetschen die Rollen 73 die Quetschkanäle 94 - wobei bei der oberen Pumpe (z.B. der Infusionspumpe 36) ein Schnitt durch die Rolle 73 mit gequetschtem Quetschquerschnitt 94 sowie bei der unteren Pumpe (z.B. der Aspirationspumpe 52) ein Schnitt neben einer Rolle 73 und entsprechend einen ungequetschten Quetschquerschnitt 94 zeigt. Mit den Zentrierelementen 82, welche in diesem Beispiel auch die Drehachse 75 des Rollenkopfes 72 bilden wird dabei beim Klemmen der Kassette 1 auch eine Feinpositionierung der Kassette 1 im Gerät 99 bewirkt.

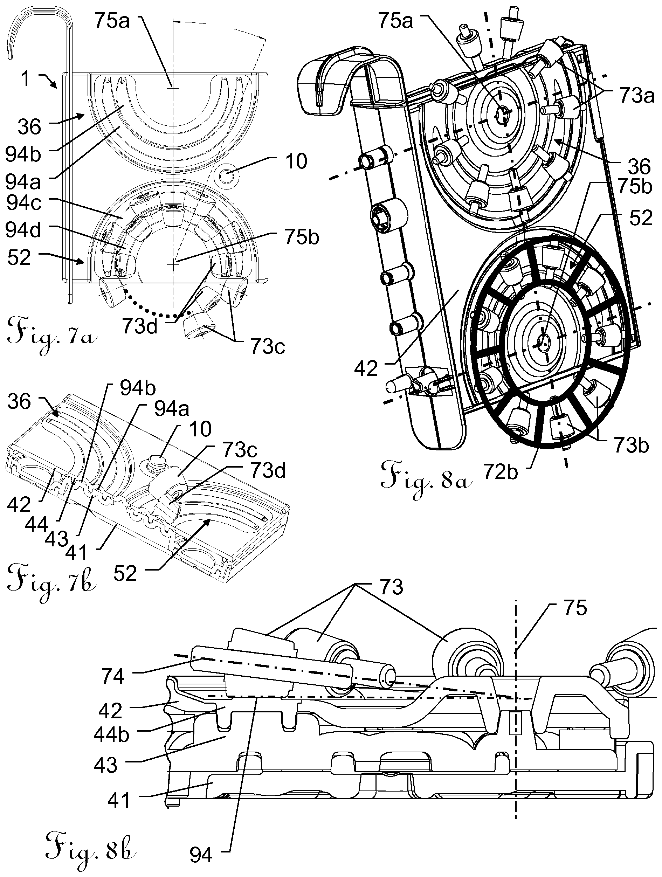

**Fig. 7a** zeigt eine beispielhafte Ausführungsform einer erfindungsgemäßen Kassette 1 in welcher eine kassettenseitige Pumpeinrichtung 36 für die erfindungsgemäße aktive Infusion sowie eine weitere Pumpeinrichtung 52 dargestellt sind.

Gezeigt sind eine erfindungsgemäße erste Pumpeinrichtung 36 für die Infusion und eine davon getrennte, zweite Pumpeinrichtung 52 für die Aspiration. Diese Pumpeinrichtungen sind mit den Weichbereichen 44 der Außenschale 42 gebildet, welche nach außen aufgewölbte Quetschkanäle 94 ausbilden. Durch den Eingriff von Rollern 73 werden diese Quetschkanäle 94 lokal abdichtend auf den Kernteil 43 gedrückt. Durch Bewegung der Roller 73 entlang der Quetschkanäle 94 kann damit ein Volumen in den Quetschkanälen 94 gefördert werden. Diese Bewegung kann hier durch eine Drehung eines Rollenkopfes 72 um die Drehachse 75b bewirkt werden, wobei der Rollenkopfe 72 die Roller 73a und 73b trägt. Speziell gezeigt sind teilweise die geräteseitigen Rollen 73c und 73d, welche sich auf konzentrischen Kreisbahnen bewegen und in die jeweils zugeordneten kassettenseitigen Quetschkanäle 94c bzw. 94d eingreifen und mit diesen zusammenwirkend die Pumpeinrichtung 52 bilden. Analoges gilt für die Pumpeinrichtung 36, wobei hier der Übersichtlichkeit halber die zu den Quetschkanälen 94a und 94b zugeordneten Roller nicht dargestellt sind.

Ein in dieser speziell weitergebildeten Ausführungsform gezeigter erfindungsgemäß ausführbarer Teilaspekt zur Verringerung von Ripple-Effekte, liegt in der dualen Pumpenausführung mit zumindest zwei benachbarten Quetschbereichen 94c und 94d. Diese sind derart ausgebildet und angeordnet, dass durch die Quetschpumpwirkung auftretende Ripple der beiden benachbarten Quetschkanäle 94c und 94d sich in Summe möglichst ausgleichen und damit der Gesamt-Ripple der Pumpe verringert wird. Dies kann beispielsweise wie gezeigt durch gegeneinander versetzte Quetschrollen 73c und 73d bewirkt werden, mit welchen eine Phasenverschiebung der von den beiden Quetschkanälen 94c und 94d jeweils erzeugten Druck- bzw. Fördermengen-Ripple bewirkt wird. Dabei sind die beiden Einlässe sowie die beiden Auslässe der Quetschkanälen 94c und 94d jeweils hydraulisch miteinander verbunden. Insbesondere kann eine Phasenverschiebung der Ripple-Verläufe um ca. 180 Grad bei zwei Kanälen, eine vorteilhafte Reduktion bewirken. Bei einer anderen Anzahl von Quetschkanälen 94 ist die Phasenverschiebung entsprechend anders zu wählen. Alternativ oder zusätzlich kann dabei auch die Lage und/oder Formgebung der Ein- und/oder Auslässe der beiden Quetschbereiche 94 unterschiedlich gestaltet werden, um den besagten Phasenversatz und/oder eine weitere Ripple-Reduktion zu bewirken. Somit kann entsprechend einer derartig weitergebildeten Ausführungsform bei entsprechender Auslegung eine Ripple-Reduktion gegenüber einem einzelnen Quetschbereich erzielt wird.

Bei der hier gezeigten, zumindest annährend konzentrischen Anordnung von zwei zusammengeschalteten Quetschkanälen 94c und 94d, kann dabei zudem berücksichtigt werden, dass mit deren unterschiedlichen Bogenradien auch unterschiedliche Bogenlängen auftreten. Zur Ripple-Kompensation können daher erfindungsgemäß die Flüssigkeitskanal-Querschnittsdimensionen, Querschnittsformen und/oder Querschnittsverläufe des äußeren 94c gegenüber dem innerer 94d Bogen derart unterschiedlich ausgebildet sein, dass das pro Bewegungseinheit der Rollen 73 geförderte Volumen stets zumindest annähernd gleich bleibt - respektive die Volumina sich stets möglichst ausgleichen und in Summe einen möglichst geringen Ripple des geförderten Gesamtvolumens ergeben. Beispielsweise also etwa derart, dass über eine ganze Umdrehung des die Rollen 73 tragenden Rollenkopfes hinweg die äußere sowie die innere Quetschbahn 94c und 94d zumindest annähernd jeweils dasselbe Volumen fördern bzw. einen möglichst gegengleichen Fördervolumen-Ripple aufweisen. Durch allenfalls unterschiedliche Durchmesser der eingreifenden Rollen 73c und 73d bedingte, Unterschiedliche Quetschradien können bei diesen Überlegungen ebenfalls berücksichtigt werden.

**Fig. 7b** zeigt die Ausführungsform von Fig. 7a nochmals in einer Schnittdarstellung durch die Kassette 1. Die Flüssigkeitskanäle im Bereich der Peristaltikpumpe sind im Inneren der Kassette 1 durch den Hartkunststoff-Kernteil 43 gebildet, über welchen ein zum Äußeren der Kassette 1 hin aufgewölbter Elastomerteil 44a der Außenschale 42 einen Teilbereich des Flüssigkeitskanals ausbildet. Dieser Teilbereich, welcher auch als Quetschbereich 94 der Pumpe bezeichnet wird, ist durch eingreifende, geräteseitige Roller 73 in der Wirkart einer Peristaltikpumpe quetschbar, womit bei Bewegung der Roller 73, die im Flüssigkeitskanal befindliche Flüssigkeit oder Luft, förderbar ist. Insbesondere kann dabei erfindungsgemäß die Querschnittsfläche des dabei ausgebildeten Quetschbereichs 94 über dessen Länge variieren. Wie in diesem Beispiel einer erfindungsgemäßen Ausführungsform gezeigt, kann etwa eine Verjüngung des Querschnitts hin zum Auslass der Pumpe und/oder eine Vergrößerung oder Verkleinerung des (ungequetschten) Querschnitts beim Einlass der Pumpe ausgebildet sein. Dabei kann insbesondere mit einer Optimierung dieser Querschnittsveränderung eine Minimierung von Druck- und/oder Fördermengen-Schwankungen (auch als Ripple bezeichnet) während des Förderns erzielt werden. Ein vorteilhafter Querschnittsverlauf entlang des Quetschbereichs 94 kann beispielsweise auch mittels Simulation und/oder Versuchsreihen ermittelt werden.

**Fig. 8a** zeigt die Kassette 1 mit im Eingriff befindlichen, beispielhaften Rollenköpfen 72. Der Übersichtlichkeit halber ist nur der untere drehbaren Rollenkopf 72b, an welchem die Rollen 73b angebracht sind, in der Figur angedeutet - bei der oberen Pumpeinrichtung sind nur die Rollen 73a nicht jedoch der zugehörige Rollenkopf 72a dargestellt. Im hier gezeigten Beispiel greifen ein erster Rollenkopf 72a und ein zweiter Rollenkopf 72b der jeweiligen getrennten Pumpen für Infusion und Aspiration auf derselben Seite der Kassette 1 ein. In einer anderen Ausführungsform kann erster und zweiter Rollenkopf 72a und 72b aber auch auf gegenüberliegenden Seiten der Kassette 1 eingreifen. Insbesondere, aber nicht nötigerweise, können dabei die Drehachsen der Rollenköpfe gegenüberliegend angeordnet sein und eine zumindest annähernd gemeinsame Drehachse ausbilden, was Vorteile bezüglich der Kraftverteilung auf die Kassette 1 bringen kann.

In dieser erfindungsgemäßen Ausführungsform sind die Quetschbereiche jeweils nur Einfach, also ohne einen hydraulisch parallelen zweiten Quetschkanal wie in der vorherigen Ausführungsform aus Fig. 7a und 7b ausgeführt.

Im Beispiel der hier gezeigten erfindungsgemäßen Ausführungsform beschrieben die beiden Pumpen 36 und 52 zumindest annähernd jeweils einen Halbkreis auf der Kassette 1. Dieses ergibt auf der beispielhaft gezeigten Kassette 1 eine vorteilhafte Ausnutzung der Platzverhältnisse, speziell bei einfacher Handhabbarkeit und Herstellbarkeit. Eine ebenfalls ausführbare, konzentrische Anordnung der beiden Pumpen 36 und 52 wäre auch möglich, aber vergleichsweise konstruktiv aufwändiger. Alternativ kann der wulstförmig aufgewölbte Quetschbereich 94 der Pumpe 36 und/oder 52 in einer anderen Ausführungsform auch mit einer anderen, insbesondere geringeren Bogenlänge ausgebildet sein, wobei jedoch stets eine entsprechende Anordnung und Anzahl der Rollen 73a bzw. 73b zu wählen ist, sodass ein hinreichendes Pumpverhalten sichergestellt werden kann - also insbesondere in jedem Quetschbereich 94 stets zumindest eine einzige, vorzugsweise mehr als eine einzige, Rolle 73a im Eingriff ist.

Wie in **Fig. 8b** im illustriert, kann dies beispielsweise erfindungsgemäß erzielt werden, indem die Rollenachsen 74 gegenüber deren Abrollebenen auf den Quetschkanälen 94 schräg angeordnet sind, und sich vorzugsweise im Drehzentrum 75 des die Rollen 73 tragenden Rollenkopfes 72 mit dieser Abrollebene schneiden. Die kegelstumpfförmigen Rollen 73 rollen damit optimal auf dem, die Quetschkanäle 94 bildenden Weichkunststoffbereich 44b der Außenschale 42 ab, und quetschen diesen gegen den Kernteil 43. Alternativ können aber allenfalls auch andere Geometrien genutzt werden. Die Ausformung und Anordnung erfolgt dabei vorzugsweise derart, dass der Umfang der Rolle 73a bzw. 73b an einer Stelle jeweils proportional dem Umfang, der von der Rolle an dieser Stelle beschriebenen Kreisbahn um den Drehpunkt 75a des Zentrums des Rollenkopfes 72a ist.

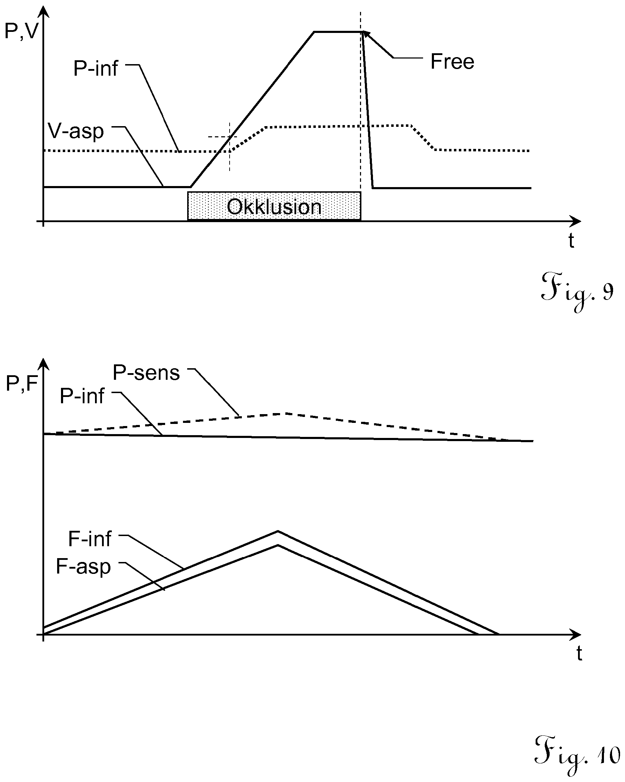

**Fig. 9** zeigt eine beispielhafte Ausführungsform eines Beispiels eines erfindungsgemäßen, voneinander abhängigen Steuerns der erfindungsgemäßen Infusions-Fördereinrichtung und Aspirations-Fördereinrichtung. Dabei ist am Anfang der "Normalzustand" gezeigt, bei welchem der Infusionsdruck P-inf zumindest annähernd konstant ist und das Aspirations-volumen V-asp ebenfalls zumindest annähernd konstant ist - also z.B. eine Aspirations-Peristaltikpumpe sich mit durchschnittlich konstanter Geschwindigkeit dreht. Als Balken auf der Abszisse, welche eine Zeitachse darstellt, ist eine Okklusion des Aspirationswerkzeugs dargestellt, also z.B. wenn ein zertrümmertes Linsenteilchen die Absaugnadelspitze verstopft. Dadurch steigt im Gerät der wie an der entsprechenden Kurve gezeigt der Aspirations-Unterdruck an. Entsprechend dem hier beschriebenen, speziellen erfindungsgemässen Teilaspekts kann nun in einem solchen Fall mit der schnellen, dynamischen steuer- bzw. Regelbarkeit des Infusionsdruckes mit der erfindungsgemässen Infusions-Fördereinrichtung, ab einem bestimmten Aspirationsunterdruck vorsorglich der Infusionsdruck erhöht werden, wie dies die entsprechende Kurve zeigt. Dies kann speziell automatisch von einer Steuereinheit im Gerät 99 erfolgen, also ohne zutun des Arztes. Dies Geschieht in Vorbereitung auf den zu erwartenden Bruch der Okklusion, also dann wenn sich die Okklusion auflöst und die Absaugnadel wieder frei wird - also am Ende des gezeigten Okklusionsbalkens. Dann kommt es nämlich aufgrund des bis dahin angestiegenen Aspirations-Unterdrucks zu einer kurzzeitigen Überhöhung der aspirierten Flüssigkeitsmenge - was zu einem (zumindest leichten) zusammenfallen des Auges führen könnte. Durch den erfindungsgemässen Teilaspekt der vorsorglichen, temporären Infusionsdruck-Erhöhung, kann dies jedoch zu einem Gutteil ausgeglichen werden, da während der (z.B. an der Aspirationsdruckerhöhung) erkannten Okklusion vorsorglich für diesen Fall bereits mehr Infusionsflüssigkeit gefördert wurde. Somit steht beim Lösen der Okklusion genügend Flüssigkeit im Auge zur Verfügung, sodass ein Kollapses (=Kollabieren der Vorderkammer) verhindert oder zumindest vermindert werden kann. Nach dem Lösen der Okklusion und wiedererreichen des Normalzustands des Aspirationsunterdrucks, kann dann auch der Infusionsdruck wieder normalisiert werden.

Fig. 10 zeigt eine beispielhafte Ausführungsform eines Beispiels einer dynamischen Anpassung des Infusionsdrucks mit der erfindungsgemässen aktiven Infusion als erfindungsgemässer Teilaspekt durchführbar ist. Gezeigt ein Diagramm in welchem, beispielhaft und auf das Grundprinzip vereinfacht, das Fördervolumen F-asp der Aspirations-Fördereinrichtung und das erfindungsgemäss abhängig davon gesteuerte Fördervolumen F-inf der aktiven Infusions-Fördereinrichtung gezeigt ist. Diese Abhängigkeit wird hier Beispielhaft derart dargestellt, dass die Infusion abhängig von einer vorgegebenen Aspiration gesteuert wird - es können im erfindungsgemässen Sinn aber ebenso Ausführungsformen implementiert werden, bei welchen die eine gewünschte Infusion vorgegeben ist und die Aspiration abhängig von dieser gesteuert ist. Im Diagramm ist die Infusionsmenge stets etwas grösser als die Aspirationsmenge, da Leckverluste bei der Öffnung in Auge kompensiert werden. Vorzugsweise wird die gezeigte Abhängigkeit von Infusion und Aspiration dabei als eine art Vorsteuerung genutzt, welcher zusätzlich eine Regelung des tatsächlich vorherrschenden Infusionsdrucks überlagert werden kann. Die Regelung wird somit vereinfacht und kann dynamischer ausgestaltet werden, da die auszugleichende Regelabweichung nur mehr gering ist. Zudem kann mit einer deutliche längeren Zeitkonstante auch die Vorsteuerung entsprechend des tatsächlich auftretenden Leckverlustes bei bedarf langsam nachgeführt und den tatsächlich bei der konkreten Operation auftretenden Verlusten angepasst werden. Es kann also beispielsweise zu Beginn des Eingriffs die Vorsteuerung mit einer für diesen Eingriff erfahrungsgemäss üblichen Leckraten-Kompensation von z.B. 7% erfolgen, welcher Prozentsatz dann z.B. auf 5% angepasst wird, falls die überlagerte Regelung über längere Zeit eine kontinuierliche Reduktion der Infusionsmenge bewirkt - die Eingriffsöffnung also dichter ist als üblicherweise angenommen.

Im oberen Teil des Diagramms ist dabei auch der zur Infusions-Fördermengen F-inf zugehörige, erfindungsgemäss angepasste Infusionsdruck P-sens am Drucksensor in der Kassette. Dabei wird mit der gezeigten Steigerung des Infusionsdrucks P-sens in der Kassette, welcher entsprechend dieses Aspekts der Erfindung abhängig von einer Erhöhung der Fördermenge F-inf durchgeführt wird, insbesondere ein fliessgeschwindigkeitsbedingter, erhöhter Druckabfall in der Leitung zum Patienten ausgeglichen und resultiert im Auge in einem ausgeglicheneren, konstanteren Infusions-Druck P-inf als im Stand der Technik. Parameter für diese fördermengenabhängige Soll-Infusionsdruckanpassung, also Beispielsweise der Kurvenverlauf der Anpassung P-sens, können beispielsweise experimentell oder rechnerisch für ein jeweils verwendetet Schlauch-Set ermittelt, gespeichert und bereitgestellt werden.

Im Stand der Technik, ohne aktive Infusion können oben genannte vorteilhafte Weiterführungen erst gar nicht in Betracht gezogen werden, da derartiges mit den Träge reagierenden Systemen des Stands der Technik nicht implementierbar ist. Zudem sind die Probleme welche diese vorteilhaften Weiterführungen lösen, im Stand der Technik auch gar nicht bekannt oder von offensichtlicher Relevanz.

Ein weiteres Problem im Stand der Technik ist, dass für die Infusion oft relative dünne Schläuche eingesetzt werden, und daher auch der Druckabfall zwischen BSS-Flasche und Gerät nicht zu vernachlässigen ist, insbesondere bei grossem Infusionsdurchfluss. Auch kommt es immer wieder vor, dass sich ein Unterdruck in der BSS-Flasche bildet, was sich im Stand der Technik negativ auf den Infusions-druck auswirkt. Auch wirkt sich Beispielsweise ein Filter in der Tropfkammer, welcher zu viel Druckabfall bewirkt negativ aus. Bei einer Schwerkraftinfusion ergibt sich in diesen Fällen kurzerhand weniger Druck im Auge und somit weniger Stabilität.

Mit der erfindungsgemässen aktiven Infusion spielt all dieses keine Rolle, da sich obige Probleme nicht, oder zumindest kaum auf den Infusionsdruck im Auge auswirken, bzw. von der aktiven Infusion aktiv ausgeglichen werden. Speziell eine Druckmessung auf der Patientenseite der Flüssigkeitsfördereinrichtung gemeinsam mit einer Druckreglung via Peristaltikpumpe, können oben genannte Druckverluste kompensieren. Ferner kann die Peristaltikpumpe aus Richtung der BSS-Flasche auch saugend wirken, womit die Durchflussmenge auch bei hohem Druckabfall in Leitung, Tropfkammer, Infusionsflasche, etc. kompensiert werden kann. Erfindungsgemäss können also - falls erforderlich - auch höhere Durchflusswerte ab der BSS-Flasche realisiert werden als nur mit einer Schwerkraftinfusion, was in manchen Operationssituationen sehr hilfreich sein kann. Speziell kann dabei schnell und dynamisch auf allfällige Veränderungen reagiert werden, was die Operation sicherer gestaltet. Insbesondere kann erfindungsgemäss eine derartige schnelle und dynamische Reaktion auf allfällige Störungen erfindungsgemäss auch automatisch, also insbesondere ohne oder mit nur geringem eingreifen des Personals, durch das Gerät ausgebildet werden. Speziell über ein Controllermodul zur Ansteuerung der Infusions-Flüssigkeitsfördereinrichtung, welches auf Basis der Sensorik der Wechselkassette vorgegebene Sollwerte und/oder Sollwertprofile des Infusionsdrucks automatisch einhält oder abfährt.

## Patentansprüche

1. Ophthalmologisches Gerät (99) welches eine Saugspülung mit einer Aspirations-Funktion und einer Infusions-Funktion bereitstellt, mit:
▪ einer Aspirations-Fördereinrichtung (52) zur motorisch angetrieben Abführung von Flüssigkeit von einem chirurgischen Instrument (29) in einen Abfallbehälter (56), insbesondere in Form einer Peristaltik-Pumpe oder einer Venturi-Aspiration mit Unterdruck-Absaugung,
▪ eine Infusions-Fördereinrichtung (36) zur motorisch angetriebenen Zuführung eines Infusionsmediums von einem Infusionsbehälter (30) über einen Infusions-Anschluss (38) und eine Leitung zum chirurgischen Instrument (29),
**dadurch gekennzeichnet, dass**
die Infusions-Fördereinrichtung (36) als Peristaltikpumpe zur aktiven Volumen-Förderung des Infusionsmediums mit einem bekannten Fördervolumen ausgebildet ist, und zwischen Infusions-Fördereinrichtung (36) und dem chirurgischen Instrument (29) eine Druckmesseinrichtung (37) mit einem Druck- oder Kraftsensor im Gerät ausgebildet ist, mittels welchem ein Infusionsdruck des Infusionsmediums im Gerät bestimmbar ist.

2. Ophthalmologisches Gerät (99) nach Anspruch 1, **dadurch gekennzeichnet, dass**
ein Steuerungsmodul im Gerät (99) derart ausgebildet ist, dass eine Regelung des Fördervolumens der Infusions-Volumen-Förderung anhand des Infusionsdrucks erfolgt.

3. Ophthalmologisches Gerät (99) nach Anspruch 2, **dadurch gekennzeichnet, dass**
Das Steuerungsmodul im Gerät (99) derart ausgebildet ist, dass die Regelung unter Berücksichtigung eines Druckabfalls in der Leitung hin zum chirurgischen Instrument (29) erfolgt, wobei der Druckabfall mit einer Drucküberhöhung bei der Infusions-Volumen-Förderung zumindest teilweise kompensiert wird.

4. Ophthalmologisches Gerät (99) nach Anspruch 3, **dadurch gekennzeichnet, dass**
das Steuerungsmodul im Gerät (99) derart ausgebildet ist, dass eine Bestimmung des Druckabfalls unter Berücksichtigung einer Fliessgeschwindigkeit des Infusionsmediums erfolgt, welche Fliessgeschwindigkeit anhand des bekanten Fördervolumens der Infusions-Volumen-Förderung ermittelt wird.

5. Ophthalmologisches Gerät (99) nach einem der Ansprüche 1 bis 4,
**dadurch gekennzeichnet, dass**
mit dem Infusionsmedium in Kontakt tretende Bereiche der Infusions-Fördereinrichtung (36) mit den mit Flüssigkeit in Kontakt tretenden Bereichen der Aspirations-Fördereinrichtung (52) in einer gemeinsamen Wechselkassette (1) für das Gerät (99) ausgebildet sind, und das Gerät (99) einen Infusions-Antrieb (76) für die Infusions-Fördereinrichtung (36) und einen davon getrennten Aspirations-Antrieb (77) für die Aspirations-Fördereinrichtung (52) aufweist.

6. Ophthalmologisches Gerät (99) nach einem der Ansprüche 1 bis 5,
**dadurch gekennzeichnet, dass**
die Druckmesseinrichtung (37) mit einer flexiblen Membrane (37) in der Wechselkassette (1) und dem Druck- oder Kraftsensor im Gerät (99) ausgebildet ist.

7. Ophthalmologisches Gerät (99) nach einem der Ansprüche 1 bis 6,
**dadurch gekennzeichnet, dass**
eine Reglereinheit im Gerät (99) derart ausgebildet ist, dass mit einer automatischen Ansteuerung der ermittelte Infusionsdruck der Infusions-Fördereinrichtung (36) auf einen Sollwert ausregelbar ist, wobei eine fördermengenabhängige Anpassung des Sollwerts erfolgt, insbesondere derart, dass ein fliessgeschwindigkeitsbedingter, erhöhter Druckabfall in der Leitung zum chirurgischen Instrument (29) ausgeglichen wird.

8. Ophthalmologisches Gerät (99) nach einem der Ansprüche 1 bis 7,
**dadurch gekennzeichnet, dass**
dieses mit einer motorisch angetriebene Höhenverstellung (32) des Infusionsbehälters (30) ausgebildet ist,
und mit einer Bypass-Ventileinrichtung (35) eine Umschaltbarkeit zur optionalen Umgehung der Infusions-Fördereinrichtung (36) bereitgestellt wird.

9. Ophthalmologisches Gerät (99) nach einem der Ansprüche 1 bis 8,
**dadurch gekennzeichnet, dass**
die Aspirations-Fördereinrichtung (52) und die Infusions-Fördereinrichtung (36) in ihrer Ausgestaltung und Dimensionierung gleichartig - insbesondere gleich - aufgebaut sind.

10. Wechselkassette (1) für ein ophthalmologisches Gerät (99) nach Anspruch 5 - mit:
▪ zumindest einem rigiden Hartteil (41,42,43) als Gehäuse der Wechselkassette (1)
▪ zumindest einem elastischen Weichteil (44)
wobei Hartteil (41,42,43) und Weichteil (44) interne Flüssigkeitskanäle der Wechselkassette (1) ausbilden und das Weichteil (44) partiell vom äußeren der Wechselkassette (1) zugänglich ist, und
wobei Bereiche des Weichteils (44) kassettenseitige Peristaltikpumpen-Quetschkanäle (94) zur Förderung von eines Infusionsmediums in zumindest einem der Flüssigkeitskanäle ausbilden, und
wobei Bereiche des Weichteils (44) zumindest einen kassettenseitigen Drucksensorbereich (37) zur Bestimmung eines Flüssigkeitsdruckes für das Infusionsmedium in zumindest einem der Flüssigkeitskanäle ausbilden.

11. System aus einem ophthalmologischen Gerät (99) nach einem der Ansprüche 1 bis 9 sowie aus einer Wechselkassette (1) nach Anspruch 10.

12. Verfahren zur automatischen Infusionsdruckregelung in einem ophthalmologischen Gerät (99), gemäss Anspruch 1, zur Saugspülung, mit
einer aktiven Aspirations-Förderung einer Aspirationsflüssigkeit von einem chirurgischen Instrument (29) hin einem Abfallbehälter (56), und
einer aktiven Infusions-Volumen-Förderung eines Infusionsmediums von einem Infusionsbehälter (30) hin zum einem Infusionsanschluss (38), mit einer Messung eines Infusionsdrucks des Infusionsmediums im Gerät (99) und
einer Regelung eines Förder-Volumens der Infusions-Volumen-Förderung anhand des Infusionsdrucks,
wobei Infusions-Volumen-Förderung und Aspirations-Förderung jeweils mittels eines peristaltischen Pumpens erfolgt.

13. Verfahren nach Anspruch 12, **dadurch gekennzeichnet, dass** die Regelung mit einer Kompensation eines Druckabfalls in einer am Infusionsanschluss angeschlossenen Leitung hin zum Auge erfolgt, wobei dieser Druckabfall anhand einer Fliessgeschwindigkeit des Infusionsmediums, welche aus dem Förder-Volumen ermittelt wird, bestimmt wird.

14. Verfahren nach einem der Ansprüche 12 bis 13, **gekennzeichnet durch** ein Abstimmen des Förder-Volumens und eines Förder-Druckes der aktiven Infusion zu einem Kennwert der aktiven Aspiration mit einer Abhängigkeit einer Bewegung einer Infusions-Peristaltikpumpe von einem Aspirations-Förder-Volumen und von einem Aspirations-Förder-Druck der aktiven Aspiration, insbesondere wobei ein Bestimmen eines Aspirationsunterdrucks erfolgt und bei einem Anstieg des Aspirationsunterdrucks ein temporäres Erhöhen des Infusionsdrucks erfolgt.

15. Verfahren zur automatischen Ansteuerung einer aktiven Infusion in einem ophthalmologischen Gerät (99) gemäss Anspruchs 1 zur Saugspülung durch eine Steuerungseinheit mit:
einem Ermitteln einer Aspirations-Fördermenge einer Aspiration der Saugspülung,
einem Vorsteuern einer Infusions-Fördermenge der aktiven Infusion zum Ausgleich der Aspirations-Fördermenge,
einem Bestimmen eines Infusionsdrucks der aktiven Infusion im Gerät (99) und
einem Nachregeln der vorgesteuerten Infusions-Fördermenge zur Erzielung eines gewünschten Infusionsdrucks, welches Nachregeln abhängig von der Infusions-Fördermenge erfolgt, insbesondere zum Ausgleich eines Druckabfalls in einer Leitung zu einem chirurgischen Instrument.

## Claims

1. Ophthalmological device (99) which provides suction flushing with an aspiration function and an infusion function, comprising
• an aspiration conveyor device (52) for the motor-driven discharge of liquid from a surgical instrument (29) into a waste container (56), in particular in the form of a peristaltic pump or a Venturi aspiration with vacuum suction,
• an infusion conveyor device (36) for the motor-driven feeding of an infusion medium from an infusion container (30) via an infusion connection (38) and a line to the surgical instrument (29),
**characterized in that**
the infusion conveyor device (36) is designed as a peristaltic pump for the active volume delivery of the infusion medium with a known delivery volume, and a pressure measuring device (37) with a pressure or force sensor in the device is formed between the infusion conveyor device (36) and the surgical instrument (29), by means of which an infusion pressure of the infusion medium in the device can be determined.

2. Ophthalmological device (99) according to claim 1, **characterized in that** a control module in the device (99) is designed such that the delivery volume of the infusion volume delivery is regulated on the basis of the infusion pressure.

3. Ophthalmological device (99) according to claim 2, **characterized in that** the control module in the device (99) is designed such that the regulation is carried out under consideration of a pressure drop in the line towards the surgical instrument (29), wherein the pressure drop is at least partially compensated with a pressure increase in the infusion volume delivery.

4. Ophthalmological device (99) according to claim 3, **characterized in that** the control module in the device (99) is designed such that the pressure drop is determined by taking into account a flow velocity of the infusion medium, which flow velocity is determined on the basis of the current delivery volume of the infusion volume delivery.

5. Ophthalmological device (99) according to one of claims 1 to 4, **characterized in that** regions of the infusion conveyor device (36) that come into contact with the infusion medium together with regions of the aspiration conveyor device (52) that come into contact with the liquid are formed in a common interchangeable cassette (1) for the device (99), and the device (99) comprises an infusion drive (76) for the infusion conveyor device (36) and an aspiration drive (77) separate therefrom for the aspiration conveyor device (52).

6. Ophthalmological device (99) according to one of claims 1 to 5, **characterized in that** the pressure measuring device (37) is designed with a flexible membrane (37) in the cassette (1) and the pressure or force sensor in the device (99).

7. Ophthalmologic device (99) according to one of claims 1 to 6, **characterized in that** a control unit in the device is designed such that the determined infusion pressure of the infusion conveyor device (36) can be adjusted to a target value by means of an automatic control, wherein an adjustment of the target infusion pressure which is dependent on the delivery rate is carried out in such a way in particular that an increased pressure drop caused by the flow velocity in the line to the surgical instrument (29) is compensated.

8. Ophthalmological device (99) according to one of claims 1 to 7, **characterized in that** said device is formed with a motor-driven height adjustment (32) of the infusion container (30), and switchability for optionally bypassing the infusion conveyor device (36) is provided by a bypass valve device (35).

9. Ophthalmological device (99) according to one of claims 1 to 8, **characterized in that**
the aspiration conveyor device (52) and the infusion conveyor device (36) are constructed in a similar - in particular identical - manner in their design and dimensioning.

10. Interchangeable cassette (1) for an ophthalmological device (99) according to claim 5, comprising:
• at least one rigid hard part (41,42,43) as the housing of the interchangeable cassette (1),
• at least one elastic soft part (44),
wherein the hard part (41, 42, 43) and the soft part (44) form internal liquid channels of the interchangeable cartridge (1) and the soft part (44) is partially accessible from the exterior of the interchangeable cassette (1), and
wherein regions of the soft part (44) form peristaltic pump squeezing channels (94) on the cassette side for conveying an infusion medium in at least one of the liquid channels, and
wherein regions of the soft part (44) form at least one pressure sensor region (37) on the cassette side for determining a liquid pressure for the infusion medium in at least one of the liquid channels.

11. System consisting of an ophthalmologic device (99) according to one of claims 1 to 9 and an interchangeable cassette (1) according to claim 10.

12. Method for the automatic infusion pressure regulation in an ophthalmological device (99) according to claim 1 for suction flushing, comprising
an active aspiration delivery of an aspiration liquid from a surgical instrument (29) to a waste container (56), and
an active infusion volume delivery of an infusion medium from an infusion container (30) to an infusion connection (38), comprising a measurement of an infusion pressure of the infusion medium in the device (99), and
a regulation of a delivery volume of the infusion volume delivery by means of the infusion pressure, wherein infusion volume delivery and aspiration delivery are each carried out by means of a peristaltic pump.

13. Method according to claim 12, **characterized in that**
the regulation is carried out with compensation of a pressure drop in a line connected to the infusion connection towards the eye, wherein this pressure drop is determined on the basis of a flow velocity of the infusion medium which is determined from the delivery volume.

14. Method according to one of claims 12 to 13, **characterized by**
tuning the delivery volume and a delivery pressure of the active infusion to a characteristic value of the active aspiration with a dependence of a movement of an infusion peristaltic pump on an aspiration delivery volume and on an aspiration delivery pressure of the active aspiration, in particular wherein an aspiration vacuum is determined and a temporary increase in the infusion pressure takes place when the aspiration vacuum increases.

15. Method for the automatic control of an active infusion in an ophthalmological device (99) according to claim 1 for suction flushing by a control unit, comprising:
a determination of an aspiration delivery rate of an aspiration of the suction flushing, a pilot control of an infusion delivery rate of the active infusion to compensate for the aspiration delivery rate,
a determination of an infusion pressure of the active infusion in the device (99), and a readjustment of the pilot-controlled infusion delivery rate to achieve a desired infusion pressure, which readjustment takes place as a function of the infusion delivery rate, in particular to compensate a pressure drop in a line to a surgical instrument.

## Revendications

1. Appareil d'ophtalmologie (99) fournissant une irrigation/aspiration avec une fonction d'aspiration et une fonction d'injection, comprenant :
• un dispositif d'acheminement de l'aspiration (52) pour l'évacuation sous l'action d'un moteur de liquide provenant d'un instrument chirurgical (29) vers un récipient de déchets (56), prenant en particulier la forme d'une pompe péristaltique ou d'aspiration par Venturi avec aspiration par dépression,
• un dispositif d'acheminement de l'injection (36) pour amener sous l'action d'un moteur un fluide à injecter d'un récipient d'injection (30) à l'instrument chirurgical (29) via un raccord d'injection (38) et une conduite,
**caractérisé en ce que** le dispositif d'acheminement de l'injection (36) est conçu comme une pompe péristaltique pour l'acheminement volumique actif du fluide à injecter avec un volume acheminé connu, et un dispositif de mesure de la pression (37) muni d'un capteur de pression ou de force, au moyen duquel une pression d'injection d'un fluide à injecter peut être déterminée dans l'appareil, est formé dans l'appareil entre le dispositif d'acheminement de l'injection (36) et l'instrument chirurgical (29).

2. Appareil d'ophtalmologie (99) selon la revendication 1, **caractérisé en ce qu'**un module de commande de l'appareil (99) est conformé pour réguler le volume acheminé par l'acheminement du volume à injecter à l'aide de la pression d'injection.

3. Appareil d'ophtalmologie (99) selon la revendication 2, **caractérisé en ce que** le module de commande de l'appareil (99) est conformé de telle manière que la régulation soit effectuée en tenant compte d'une baisse de pression dans la conduite menant à l'instrument chirurgical (29), laquelle baisse de pression est au moins partiellement compensée par une augmentation de la pression lors de l'acheminement du volume à injecter.

4. Appareil d'ophtalmologie (99) selon la revendication 3, **caractérisé en ce que** le module de commande de l'appareil (99) est conformé de telle manière qu'une baisse de pression est déterminée en tenant compte d'une vitesse d'écoulement du fluide à injecter, laquelle vitesse d'écoulement est déterminée à l'aide du volume connu acheminé par l'acheminement du volume à injecter.

5. Appareil d'ophtalmologie (99) selon l'une des revendications 1 à 4, **caractérisé en ce que** les zones du dispositif d'acheminement de l'injection (36) venant en contact avec le fluide à injecter sont formées avec les zones venant en contact avec du liquide du dispositif d'acheminement de l'aspiration (52) dans une cassette interchangeable (1) commune destinée à l'appareil (99) et l'appareil (99) comporte un entraînement d'injection (76) pour le dispositif d'acheminement de l'injection (36) et un entraînement d'aspiration (77) séparé de celui-ci pour le dispositif d'acheminement de l'aspiration (52).

6. Appareil d'ophtalmologie (99) selon l'une des revendications 1 à 5, **caractérisé en ce que** le dispositif de mesure de la pression (37) est formé d'une membrane (37) flexible dans la cassette interchangeable (1) et du capteur de pression ou de force dans l'appareil (99).

7. Appareil d'ophtalmologie (99) selon l'une des revendications 1 à 6, **caractérisé en ce qu'**une unité de régulation est formée dans l'appareil (99) de telle manière que la pression d'injection déterminée du dispositif d'acheminement de l'injection (36) peut être réglée à une valeur de consigne avec une activation automatique, la valeur de consigne étant adaptée en fonction du débit d'acheminement, en particulier de manière à compenser une baisse de pression accrue due à la vitesse d'écoulement dans la conduite menant à l'instrument chirurgical (29).

8. Appareil d'ophtalmologie (99) selon l'une des revendications 1 à 7, **caractérisé en ce qu'**il est muni d'un réglage en hauteur (32) entraîné par un moteur pour le récipient d'injection (30) et un dispositif de valve de dérivation (35) permet une commutation pour le contournement optionnel du dispositif d'acheminement de l'injection (36).

9. Appareil d'ophtalmologie (99) selon l'une des revendications 1 à 8, **caractérisé en ce que** le dispositif d'acheminement de l'aspiration (52) et le dispositif d'acheminement de l'injection (36) ont une conception et un dimensionnement similaires, en particulier identiques.

10. Cassette interchangeable (1) pour un appareil d'ophtalmologie (99) selon la revendication 5, avec :
• au moins une partie dure rigide (41, 42, 43) formant le boîtier de la cassette interchangeable (1),
• au moins une partie souple élastique (44),
dans laquelle la partie dure (41, 42, 43) et la partie souple (44) forment des conduits de liquide internes de la cassette interchangeable (1) et la partie souple (44) est partiellement accessible par l'extérieur de la cassette interchangeable (1) et
dans laquelle des zones de la partie souple (44) forment des conduits compressibles de pompe péristaltique (94) du côté de la cassette pour l'acheminement d'un fluide à injecter dans au moins un des conduits de liquide, et
dans laquelle des zones de la partie souple (44) forment au moins une zone de capteur de pression (37) du côté de la cassette pour déterminer une pression de liquide pour le fluide à injecter dans au moins un des conduits de liquide.

11. Système composé d'un appareil d'ophtalmologie (99) selon l'une des revendications 1 à 9 et d'une cassette interchangeable (1) selon la revendication 10.

12. Procédé pour la régulation automatique de la pression d'injection dans un appareil d'ophtalmologie (99) selon la revendication 1 pour l'irrigation/aspiration, avec un acheminement actif de l'aspiration d'un liquide aspiré allant d'un instrument chirurgical (29) vers un récipient de déchets (56) et
avec un acheminement actif du volume à injecter d'un fluide à injecter allant d'un récipient d'injection (30) vers un raccord d'injection (38), avec une mesure d'une pression d'injection du fluide à injecter dans l'appareil (99) et
avec une régulation d'un volume acheminé de l'acheminement du volume à injecter basée sur la pression d'injection,
dans lequel l'acheminement du volume à injecter et l'acheminement de l'aspiration sont effectués chacun au moyen d'un pompage péristaltique.

13. Procédé selon la revendication 12, **caractérisé en ce que** la régulation est effectuée avec une compensation d'une baisse de pression dans une conduite raccordée au raccord d'injection jusqu'à l'oeil, cette baisse de pression étant déterminée à l'aide d'une vitesse d'écoulement du fluide à injecter, qui est déterminée à partir du volume acheminé.

14. Procédé selon l'une des revendications 12 à 13, **caractérisé en ce que** le volume acheminé et une pression d'acheminement de l'injection active sont ajustés à une valeur caractéristique de l'aspiration active avec une dépendance entre un mouvement d'une pompe péristaltique d'injection et un volume acheminé d'aspiration et une pression d'acheminement d'aspiration de l'aspiration active, une dépression d'aspiration étant en particulier déterminée et la pression d'injection étant temporairement augmentée en cas d'augmentation de la dépression d'aspiration.

15. Procédé pour l'actionnement automatique d'une injection active dans un appareil d'ophtalmologie (99) selon la revendication 1 pour l'irrigation/aspiration par une unité de commande, comprenant :
une détermination d'un débit d'acheminement d'aspiration d'une aspiration de l'irrigation/aspiration,
une commande en amont d'un débit d'acheminement d'injection de l'injection active pour compenser le débit d'acheminement d'aspiration,
une détermination d'une pression d'injection de l'injection active dans l'appareil (99) et
une régulation a posteriori du débit d'acheminement d'injection réglé en amont afin d'obtenir une pression d'injection souhaitée, laquelle régulation a posteriori est effectuée en fonction du débit d'acheminement d'injection, en particulier dans le but de compenser une baisse de pression dans une conduite menant à un instrument chirurgical.
